# EUROPEAN PATENT APPLICATION

(11) **EP 4 471 044 A1**
(43) Date of publication of application: **04.12.2024**
(21) Application number: 22923673.2
(22) Date of filing: 30.12.2022
(51) Int. Cl.: C07J 9/00

(54) **METHOD FOR SYNTHESIZING HIGH-PURITY PLANT-DERIVED CHOLESTEROL**

(30) Priority: 27.01.2022 CN 202210099911
(71) Applicant: East China Normal University, Shanghai 200241 (CN); Jiangsu Jiaerke Pharmaceuticals Group Corp., Ltd., Changzhou, Jiangsu 213111 (CN)
(72) Inventor: QIU, Wenwei, Shanghai 200241 (CN); GU, Xiangzhong, Jiangsu 213111 (CN); LI, Chenchen, Shanghai 200241 (CN); JIANG, Chengyu, Jiangsu 213111 (CN); YE, Rufei, Jiangsu 213111 (CN)
(74) Representative: Barker Brettell LLP
(86) International application number: PCT/CN2022/144011
(87) International publication number: WO 2023/142888

(57) **Abstract**

Disclosed in the present invention is a method for synthesizing high-purity plant-derived cholesterol. By taking plant-derived 21-hydroxy-20-methylpregn-4-en-3-one, also called bisnoralcohol or BA, as a raw material, cholesterol is synthesized by means of steps such as oxidation, Wittig reaction, acetylation, reduction and selective hydrogenation reduction, or the cholesterol is synthesized by means of steps such as oxidation, Wittig reaction, acetylation, reduction, hydroxyl protection, selective hydrogenation reduction, deprotection or hydrolysis, and the purity can reach 99% or above. For the defect that there is cholesterol in a conventional animal source, according to the present invention, a plant-derived raw material, namely BA is used for synthesizing the cholesterol, such that the safety is high, the risk of pathogenic bacteria and virus infection is avoided, the synthesis yield is high, the product purity is good, the environment is friendly, and industrial production is facilitated; moreover, the present invention greatly reduces the impurity level in the product, facilitates the obtaining of high-purity cholesterol, and improves the safety of clinical use.

## Description

### TECHNICAL FIELD

The invention belongs to the technical field of organic chemical synthesis, and relates to a method for synthesizing cholesterol with plant-derived 21-hydroxyl-20-methylpregn-4-en-3-one, also known as bisnoralcohol or BA (Bisnoralcohol) as raw material.

### BACKGROUND OF THE INVENTION

Cholesterol, is an indispensable and important substance in animal tissue cells, which not only participates in the formation of cell membranes, but also is a raw material for the synthesis of bile acids and steroid hormones in the body. At present, commercial cholesterol is mainly used for: 1. Pharmaceutical excipients-liposome additives, such as auxiliary materials for novel coronavirus mRNA vaccine (adding 30-50% cholesterol when preparing liposomes can greatly improve the drug loading and stability of liposomes); 2. cosmetic additives; 3. raw materials for the production of liquid crystals; 4. starting materials for the production of vitamin D3. At present, most of the commercial cholesterol comes from animal brainstem (brainstem cholesterol) or lanolin (lanolin cholesterol), both of which are animal-derived cholesterol. Studies have found that animal-derived products are likely to carry animal pathogens or other harmful factors, especially with the occurrence of mad cow disease, streptococcus suis, avian influenza and other infections, making people pay more and more attention to the safety of cholesterol. For example, if the upstream raw material for the production of vitamin D3 is cholesterol, due to the risk of epidemics, European and American countries have long prohibited the use of brainstem cholesterol as a raw material, and China has also restricted the use of brainstem cholesterol as a raw material since July 1, 2020. Therefore, in order to ensure people's health and safety, it is urgent to develop a plant-derived, high-purity synthetic method for cholesterol.

The chemical synthesis of cholesterol mainly include the following methods:
(1) Cholesterol was synthesized with a total molar yield of 61% using diosgenin as a raw material through 6 steps of reaction (CN 1772760 A, as shown in Scheme 1). The raw material price of this route is relatively high, the steps are cumbersome, the reagents used are highly toxic and pollute, and are not suitable for industrial production. 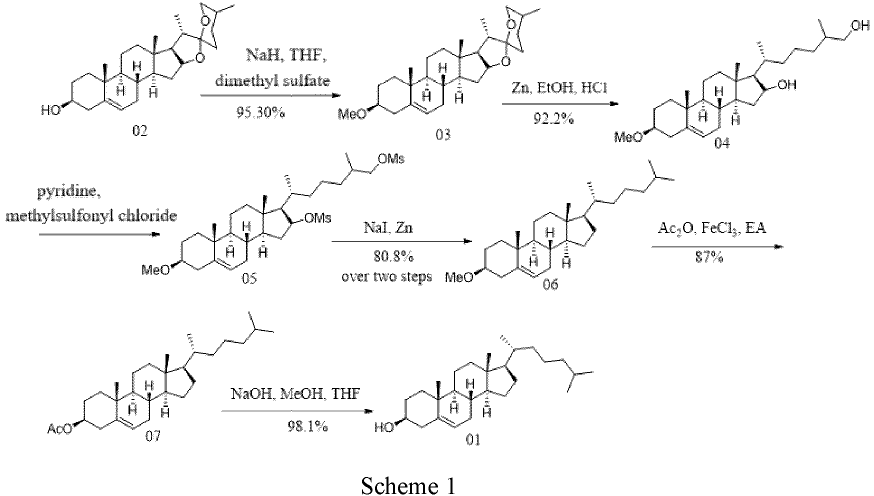
(2) Using stigmasterol degradation products as raw materials, cholesterol was synthesized with a total molar yield of 67% through 5 steps of reaction (CN 105218610 A, as shown in Scheme 2). However, the applicant of the present invention has shown the following problems in this route through experiments: when triethyl orthoformate is used for etherification protection of C-3 carbonyl group in the first step of the patent, the selectivity is poor and the side chain aldehyde group is easily reacted to form acetals (for details, see "Comparative Example 1" in the Summary of the Invention). Therefore, there are serious problems with the feasibility of this route. 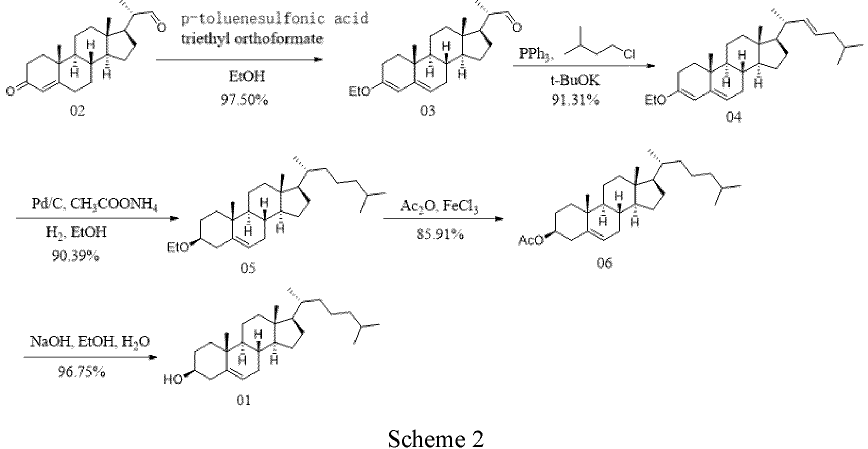
(3) Using pregnenolone as a raw material, cholesterol was synthesized through 4 steps of reaction with a total molar yield of 72% (CN 105218609 A, as shown in Scheme 3), this route used expensive rhodium catalyst and chiral phosphine ligands, and not suitable for large-scale industrial production. 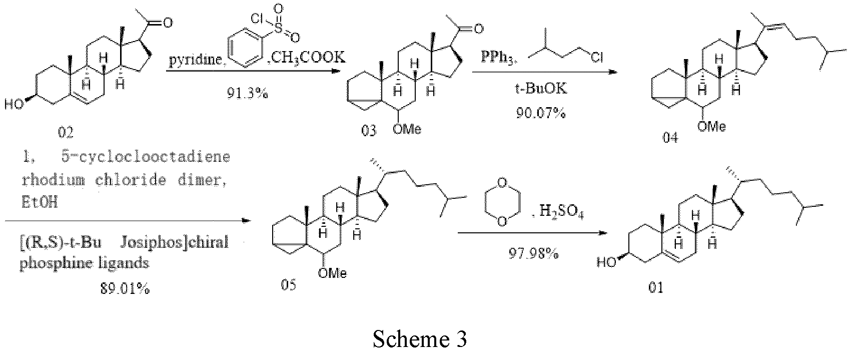
(4) Using pregnenolone as a raw material, through 2 steps of reaction, synthesize cholesterol with a total molar yield of 80%, (CN 104961788 A, as shown in Scheme 4), this route also uses noble metal rhodium catalyst and chiral Phosphine ligands, which are expensive and not suitable for large-scale industrial production. 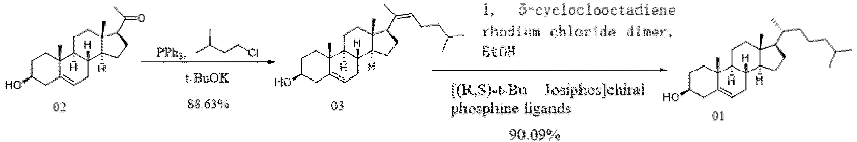 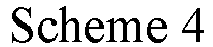
(5) Using stigmasterol as a raw material, through 5 steps of reaction, synthesize cholesterol with a total molar yield of 68%, (CN 105237603 A, as shown in Scheme 5), this route uses O₃ in the synthesis process, which requires higher monitoring of the reaction and equipment, but its economy and safety are not good enough. 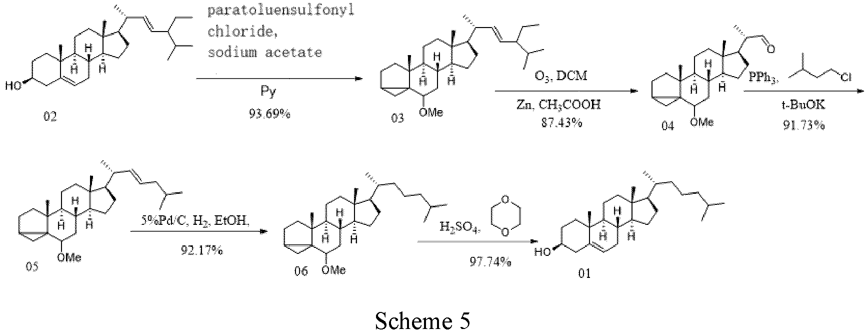
(6) Using stigmasterol as a raw material, through 4 steps of reaction, synthesize cholesterol with a total molar yield of 70%, (CN 106632565 A, as shown in Scheme 6), this route also uses O₃, which increases the difficulty of the process, higher requirements are placed on the monitoring response and equipment, and the economy and safety are not good.

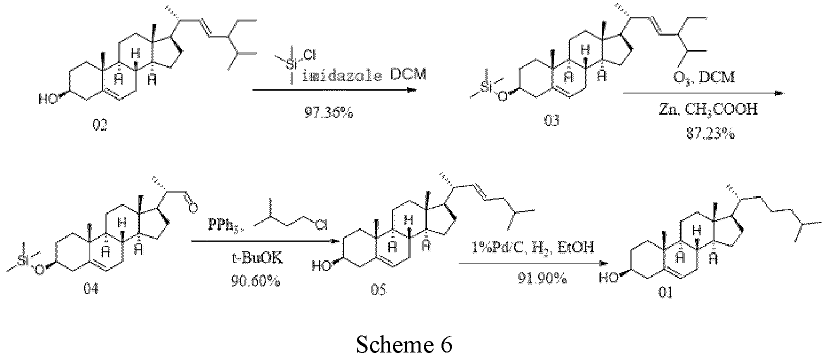

Cholesterol from animal sources has the risk of infection such as mad cow disease, streptococcus suis, and bird flu. The currently reported chemical synthesis routes of cholesterol have disadvantages such as cumbersome operation, high pollution, expensive catalysts, and low product purity. Therefore, it is of great value to develop a safer and more efficient high-purity cholesterol synthesis method based on plant-derived raw materials.

### SUMMARY OF THE INVENTION

In order to solve the shortcomings of the prior art, the object of the present invention is to provide a method for synthesizing high-purity plant-derived cholesterol. The present invention uses plant-derived 21-hydroxy-20-methylpregn-4-en-3-one, also known as bisnoralcohol or BA, as raw material to synthesize the cholesterol through oxidation, Wittig reaction, acetylation, reduction, selective hydrogenation reduction, etc; or the cholesterol can be synthesized by oxidation, Wittig reaction, acetylation, reduction, hydroxyl protection, selective hydrogenation reduction, deprotection or hydrolysis, and the purity can reach more than 99%. The starting material BA selected for synthesizing cholesterol in the present invention is safe and economical, and the method for synthesizing cholesterol is simple in operation, high in yield, good in purity, friendly in environment, and convenient for industrialized production.

The raw material BA (bisnoralcohol) used in the present invention is derived from the fermentation of phytosterols left over from the oil process, it is a green raw material of plant origin, at present, the annual output reaches 1,000 tons, and the price is cheap, and the risk of pathogenic bacteria and viral infection in animal-derived cholesterol in the prior art can be well avoided.

In the synthesis method of the present invention, the raw material BA includes, but is not limited to, biofermentation of phytosterols, or chemical synthesis.

Two kinds of methods for synthesizing cholesterol with BA raw material provided by the present invention comprise the following steps:
Method One:
   step (a), in the first solvent, the BA shown in formula (1) undergoes oxidation reaction to obtain the compound of formula (2);
   step (b), in the second solvent, the compound of formula (2) undergoes Wittig reaction to obtain the compound of formula (3);
   step (c), in the third solvent, the compound of formula (3) undergoes acetylation reaction to obtain the compound of formula (4);
   step (d), in the fourth solvent, the compound of formula (4) undergoes reduction reaction to obtain the compound of formula (5);
   step (e), in the fifth solvent, the compound of formula (5) undergoes selective hydrogenation reduction reaction to obtain cholesterol;
Method Two:
   step (a), in the first solvent, the BA shown in formula (1) undergoes oxidation reaction to obtain the compound of formula (2);
   step (b), in the second solvent, the compound of formula (2) undergoes Wittig reaction to obtain the compound of formula (3);
   step (c), in the third solvent, the compound of formula (3) undergoes acetylation reaction to obtain the compound of formula (4);
   step (d), in the fourth solvent, the compound of formula (4) undergoes reduction reaction to obtain the compound of formula (5);
   step (f), in the sixth solvent, the compound of formula (5) undergoes hydroxyl protection reaction to obtain the compound of formula (6);
   step (g), in the seventh solvent, the compound of formula (6) undergoes selective hydrogenation reduction reaction to obtain the compound of formula (7);
   step (h), in the eighth solvent, the compound of formula (7) undergoes deprotection or hydrolysis reaction to obtain cholesterol;
   wherein, the reaction process of the said method is as shown in route (A): 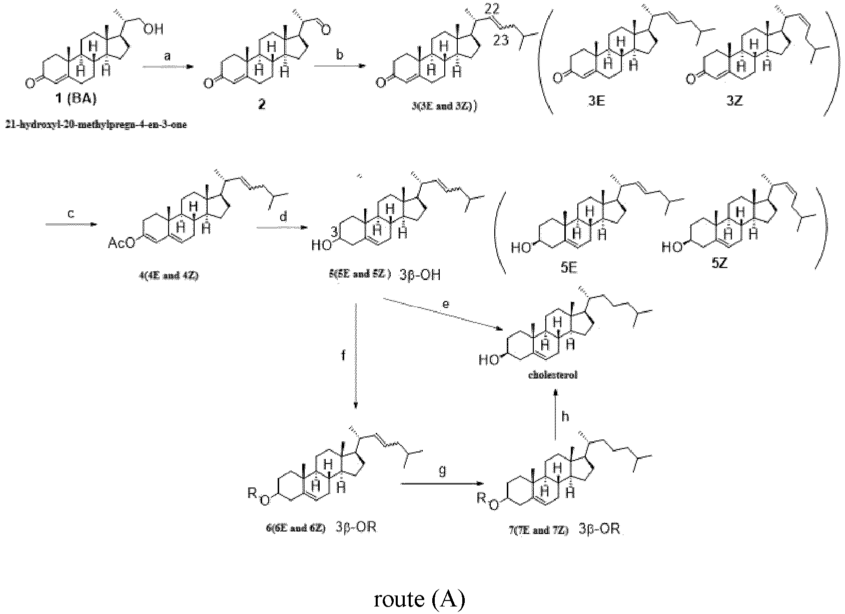
   wherein, R is selected from ester group, silicon ether group;
   preferably, R is selected from one or more of C2-C10 straight chain ester group, isobutyl ester 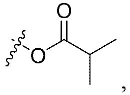 isopentyl ester 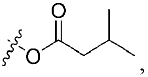 phenyl ester 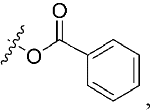 p-methoxyphenyl ester 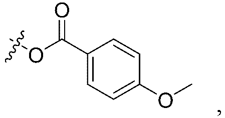 trimethylsilyl ether 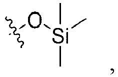 tert-butyldimethylsilyl ether 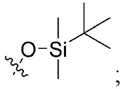
   Further preferably, R is selected from one or more of ethyl ester 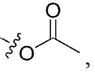 propyl ester 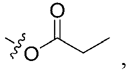 butyl ester 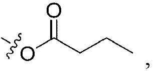 isobutyl ester 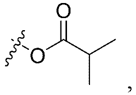 isopentyl ester 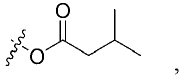 phenyl ester 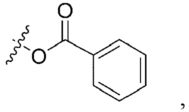 p-methoxyphenyl ester 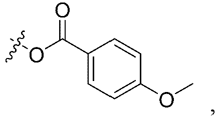 trimethylsilyl ether 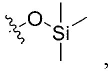 tert-butyl dimethyl silyl ether

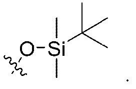

Note: In compounds of formula (3) to (6), the double bonds between C-22 and C-23 are dominated by the E configuration, supplemented by the Z configuration, and the ratio of the two configurations is E/Z≈87/13 (judged by ¹H NMR), if there is no subsequent purification operation, this ratio will remain unchanged.

In the step (a) of the present invention, the said oxidation reaction is specifically: in the first solvent, the BA shown in formula (1), TEMPO, sodium bicarbonate, tetrabutylammonium bromide, and oxidizing agent are subjected to oxidation reaction, to obtain the formula (2) compounds.

Wherein, the mol ratio of BA shown in formula (1), TEMPO, sodium bicarbonate, tetrabutylammonium bromide, and oxidizing agent is 1: (0~1): (0~20): (0~1): ( 1~5); preferably, is 1:0.01:1.35:0.1:1.15.

Wherein, the said oxidation reaction is carried out under the action of oxidizing agent, and the said oxidizing agent is selected from one or more of N-chlorosuccinimide NCS, N-bromosuccinimide NBS, 2-iodylbenzoic acid IBX, etc; preferably, is N-chlorosuccinimide NCS.

Wherein, the said first solvent is selected from one or more of dichloromethane, tetrahydrofuran, toluene, dimethyl sulfoxide, water, etc.; preferably, is a mixed solvent of dichloromethane and water (volume ratio V/ V=5/2).

Wherein, the temperature of the said oxidation reaction is 0-30°C; preferably, is 0°C.

Wherein, the time of the said oxidation reaction is 3-8 hours; preferably, is 6 hours.

In a specific embodiment, the synthesis step of the compound of formula (2) comprises: the BA shown in formula (1) is dissolved in the first solvent, then added TEMPO, sodium bicarbonate, tetrabutylammonium bromide, NCS, and subjected to oxidation reaction, to obtain the compound of formula (2).

In the step (b) of the present invention, the said Wittig reaction is specifically: 1-halo-3-methylbutane and triphenylphosphine are added into the second solvent, reflux reaction, cooling, and suction filtration to obtain the quaternary phosphonium salt; then added the said quaternary phosphonium salt and potassium tert-butoxide into the second solvent, and then added the compound of formula (2), and subjected to Wittig reaction, to obtain the compound of formula (3).

Wherein, the molar ratio of compound of formula (2), 1-halo-3-methylbutane, triphenylphosphine, and potassium tert-butoxide is 1: (1 ~ 4): (1 ~ 4): (1 ~ 4); preferably, is 1:3:2:2.

Wherein, the said second solvent is one or more of xylene, toluene, benzene, tetrahydrofuran, heptane, etc.; preferably, is xylene.

Wherein, for the synthesis of the said quaternary phosphonium salt, the preferred solvent is xylene.

Wherein, the said 1-halo-3-methylbutane is selected from one or both of 1-chloro-3-methylbutane, 1-bromo-3-methylbutane, etc.; preferably, is 1-bromo-3-methylbutane.

Wherein, the temperature of the said Wittig reaction is -10~145°C; preferably, is 10°C.

Wherein, the time of the said Wittig reaction is 0.5~24 h; preferably, is 0.5~9 h; more preferably, is 1 h.

In the step (c) of the present invention, the acetylation reaction is specifically: the compound of formula (3), acetyl chloride, acetic anhydride, and alkali are subjected to acetylation reaction in the third solvent to obtain the compound of formula (4).

Wherein, the molar ratio of the compound of formula (3), acetyl chloride, acetic anhydride, and alkali is 1:(0.5~62.5):(1~62.5):(0~6); preferably, is 1:25:24:4 .

Wherein, the said alkali is selected from one or more of pyridine, triethylamine, DIPEA, DMAP, diisopropylamine, etc.; preferably, is diisopropylamine.

Wherein, the said third solvent is one or more of acetic anhydride, acetyl chloride, ethyl acetate, dichloromethane, etc.; preferably, is a mixed solvent of acetyl chloride and acetic anhydride.

Wherein, the temperature of the said acetylation reaction is 40 ~ 110°C; preferably, is 70°C.

Wherein, the time of the said acetylation reaction is 1 ~ 10 h; preferably, is 2 ~ 10 h; more preferably, is 6 h.

Wherein, in the said acetylation reaction, acetyl chloride and acetic anhydride are both used as reactants and solvents.

In a specific embodiment, the synthesis step of the compound of formula (4) comprises: acetyl chloride, acetic anhydride and alkali are added to the compound of formula (3) and subjected to acetylation reaction to obtain the compound of formula (4).

In the step (d) of the present invention, the reduction reaction is specifically: the compound of formula (4) and the reducing agent are subjected to reduction reaction in the fourth solvent to obtain the compound of formula (5).

Wherein, the molar ratio of the compound of formula (4) and the reducing agent is 1:(1~25); preferably, is 1:4.

Wherein, the said fourth solvent is one or more of tetrahydrofuran, ethanol, water, dichloromethane, 2-methyltetrahydrofuran, isopropanol, acetic acid, methyl tert-butyl ether, etc.; preferably, is tetrahydrofuran, ethanol, water mixed solvent (volume ratio V/V/V=10/5/3).

Wherein, the said reducing agent is one or more of NaBH₄, KBH₄, etc.; preferably, is NaBH₄.

Wherein, the temperature of the said reduction reaction is 0~50°C; preferably, is 25°C.

Wherein, the time of the said reduction reaction is 6~12 hours; preferably, is 8 hours.

In a specific embodiment, the synthesis step of the compound of formula (5) comprises: the compound of formula (4) is dissolved in the fourth solvent, and subjected to reduction reaction with reducing agent to obtain the compound of formula (5).

In the step (e) of the present invention, the selective hydrogenation reduction reaction is specifically: under the action of a catalyst, the compound of formula (5) and a reducing agent are subjected to selective hydrogenation reduction reaction in the fifth solvent to obtain cholesterol.

Wherein, a purification step is also included before the cholesterol is obtained, and the said purification step is one or more of column chromatography, recrystallization, beating and the like.

Wherein, the said catalyst is Raney Ni.

Wherein, the said reducing agent is H₂.

Wherein, the mass ratio of the compound of formula (5) and the catalyst Raney Ni is 1: (0.05~5); preferably, is 1:1.

Wherein, the said fifth solvent is selected from one or more of isopropanol, dichloromethane, methanol, 2-methyltetrahydrofuran, tetrahydrofuran, ethanol, water, methyl tert-butyl ether, ethyl acetate, toluene, etc.; preferably, is isopropanol.

Wherein, the temperature of the said hydrogenation reduction reaction is 0 ~ 60°C; preferably, is 30°C.

Wherein, the pressure of the reducing agent H₂ in the hydrogenation reduction reaction is 1~20 atm, preferably, is 1 atm.

Wherein, the time of the hydrogenation reduction reaction is 6 ~ 10 hours; preferably, is 6 hours.

In a specific embodiment, the cholesterol synthesis step comprises: the compound of formula (5) is dissolved in the fifth solvent, added Raney Ni and H₂ for replacement, and subjected to selective hydrogenation reduction reaction to obtain cholesterol.

In step (f) of the present invention, when the hydroxyl protecting group R is an ester group, the hydroxyl protecting reaction is specifically: the compound of formula (5) is reacted with the reagent for protecting hydroxyl in the sixth solvent under the action of an alkali, to obtain the compound of formula (6).

The ester group is selected from one or more of C2 ~ C10 linear ester group (ethyl ester 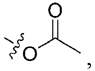 propyl ester 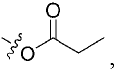 butyl ester 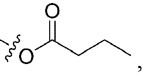 etc.), isobutyl ester 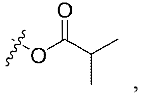 isopentyl ester 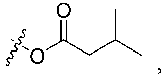 phenyl ester 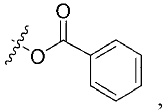 p-methoxyphenyl ester 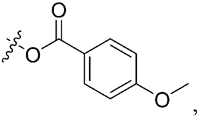 etc.; preferably, is ethyl ester.

Wherein, the molar ratio of the compound of formula (5), the reagent for protecting hydroxyl, and the alkali is 1:(1~4):(0.05~5); preferably, is 1:3:0.1.

Wherein, the said sixth solvent is one or more of ethyl acetate, dichloromethane, chloroform, DMF, toluene, tetrahydrofuran, 2-methyltetrahydrofuran, etc.; preferably, is ethyl acetate.

Wherein, the said alkali is selected from one or more of triethylamine, diisopropylethylamine, imidazole, pyridine, DMAP, etc.; preferably, is DMAP.

Wherein, the temperature of the said reaction is 0~50°C; preferably, is 45°C.

Wherein, the time of the said reaction is 2~24 hours; preferably, is 4 hours.

In the step (f) of the present invention, when the hydroxyl protecting group R is a silicon ether group, the hydroxyl protecting reaction is specifically: the compound of formula (5) is reacted with the reagent for protecting hydroxyl in the sixth solvent under the action of an alkali to obtain the compound of formula (6).

Wherein, the silicon ether group is selected from trimethylsilyl ether group 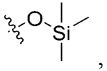 tert-butyldimethylsilyl ether group 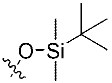 etc.; preferably, is tert-butyldimethylsilyl ether group

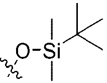

Wherein, the molar ratio of the compound of formula (5), the reagent for protecting hydroxyl, and the alkali is 1:(2~4):(4~8); preferably, is 1:2.5:4.

Wherein, the said sixth solvent is one or more of DMF, dichloromethane, chloroform, tetrachloromethane, etc.; preferably, is dichloromethane.

Wherein, the said alkali is selected from one or more of triethylamine, diisopropylethylamine, imidazole, pyridine, DMAP, etc.; preferably, is imidazole.

Wherein, the temperature of the said reaction is 0~50°C; preferably, is 25°C.

Wherein, the time of the said reaction is 2~24 hours; preferably, is 12 hours.

In a specific embodiment, the synthesis step of the compound of formula (6) comprises: the compound of formula (5) is dissolved in the sixth solvent, and reacted with a reagent for protecting hydroxyl under the action of an alkali to obtain the compound of formula (6).

In the step (g) of the present invention, the selective hydrogenation reduction reaction is specifically: under the action of a catalyst, the compound of formula (6), and reducing agent are subjected to selective hydrogenation reduction reaction in the seventh solvent to obtain the compound of formula (7).

Wherein, the said catalyst is Raney Ni.

Wherein, the said reducing agent is H₂.

Wherein, the mass ratio of the compound of formula (6) and the catalyst Raney Ni is 1: (0.05~5); preferably, is 1:1.

Wherein, the said seventh solvent is selected from one or more of 2-methyltetrahydrofuran, tetrahydrofuran, ethyl acetate, toluene, isopropanol, etc.; preferably, is ethyl acetate.

Wherein, the temperature of the said hydrogenation reduction reaction is 0 ~ 60°C; preferably, is 30°C.

Wherein, the pressure of the reducing agent H₂ in the said hydrogenation reduction reaction is 1~20 atm, preferably, is 1 atm.

Wherein, the time of the said hydrogenation reduction reaction is 4~48 hours; preferably, 4~30 hours; more preferably, is 7 hours.

In a specific embodiment, the synthesis steps of the compound of formula (7) comprises: the compound of formula (6) is dissolved in the seventh solvent, added Raney Ni and H₂ for replacement, and subjected to selective hydrogenation reduction reaction to obtain the compound of formula (7).

In step (h) of the present invention, when the hydroxyl protecting group R is an ester group, the hydrolysis reaction is specifically: under the action of an alkali, the compound of formula (7) is subjected to hydrolysis reaction in the eighth solvent to obtain cholesterol.

Wherein, the said alkali is selected from one or more of LiOH, KOH, NaOH, t-BuOK, K₂CO₃, etc.; preferably, is K₂CO₃ .

Wherein, the molar ratio of the compound of formula (7) and the alkali is 1:(0.5~2); preferably, is 1:1.3.

Wherein, the said eighth solvent is selected from one or two of methanol, ethanol, etc.; preferably, is methanol.

Wherein, the temperature of the said hydrolysis reaction is 10~75°C; preferably, is 65°C.

Wherein, the time of the said hydrolysis reaction is 0.3 ~12 hours; preferably, is 2 hours.

In the step (h) of the present invention, when the hydroxyl protecting group R is a silicon ether group, the deprotection reaction is specifically: under the action of a catalyst, the compound of formula (7) is subjected to deprotection reaction in the eighth solvent to obtain cholesterol.

Wherein, the said catalyst is selected from one or more of tetrabutylammonium fluoride TBAF, tetrabutylammonium fluoride trihydrate TBAF 3H₂O, boron trifluoride ether, acetic acid, ethyl acetate solution of hydrogen chloride, etc.; preferably, is TBAF 3H₂O.

Wherein, the mass ratio of the compound of formula (7) and the catalyst is 1:(1~6); preferably, is 1:4.

Wherein, the said eighth solvent is selected from one or both of tetrahydrofuran, water, etc.; preferably, is etrahydrofuran.

Wherein, the temperature of the said deprotection reaction is 10 ~ 75°C; preferably, is 25°C.

Wherein, the time of the said deprotection reaction is 2~48 hours; preferably, is 24 hours.

In a specific embodiment, the synthesis step of cholesterol comprises: the compound of formula (7) is dissolved in an eighth solvent, added an alkali or a catalyst, and subjected to deprotection or hydrolysis reaction to obtain cholesterol.

The present invention also provides twenty kinds of compounds, the structures of which are shown in formulas 6-2(E), 6-3(E), 6-5(E), 6-6(E), 6-2(Z ), 6-3(Z), 6-5(Z), 6-6(Z), 6'-1(E), 6'-2(E), 6'-3(E), 6'- 4(E), 6'-5(E), 6'-6(E), 6'-1(Z), 6'-2(Z), 6'-3(Z), 6'-4( Z), 6'-5(Z), 6'-6(Z):

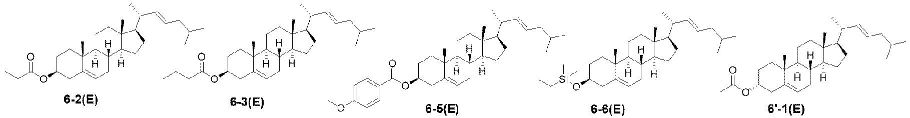

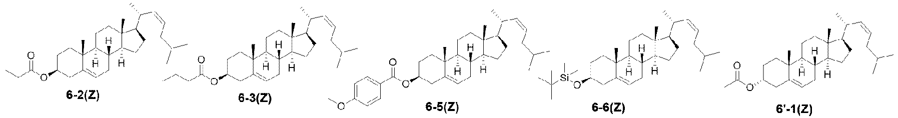

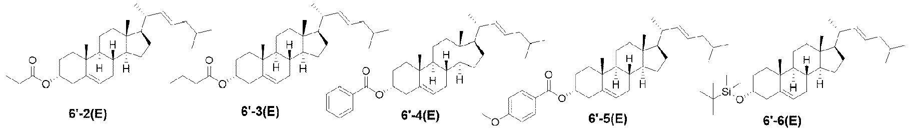

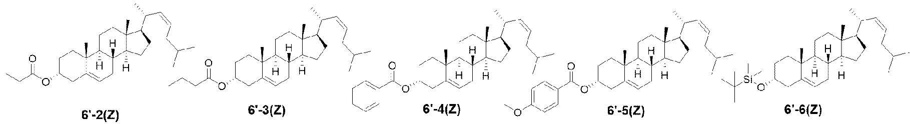

The beneficial effects of the present invention include: in the preparation method of cholesterol of the present invention, the commercial raw material BA is the plant-derived raw material, which avoids the risk of pathogenic bacteria and virus infection that may exist in animal-derived raw material, and it is cheap and easy to obtain; and the purity of cholesterol obtained is high(>99%), simple and convenient synthesis steps, high yield, few side reactions, environment-friendly, convenient to realize the industrialized production of high-purity cholesterol; solve the problems of poor safety, low purity, high synthesis cost and unfriendly environmental problems of existing cholesterol products.

### DESCRIPTION OF DRAWINGS

Figure 1 shows the gas chromatogram of cholesterol obtained in Example 5 of the present invention.
Figure 2 shows the single crystal diffraction pattern of cholesterol.

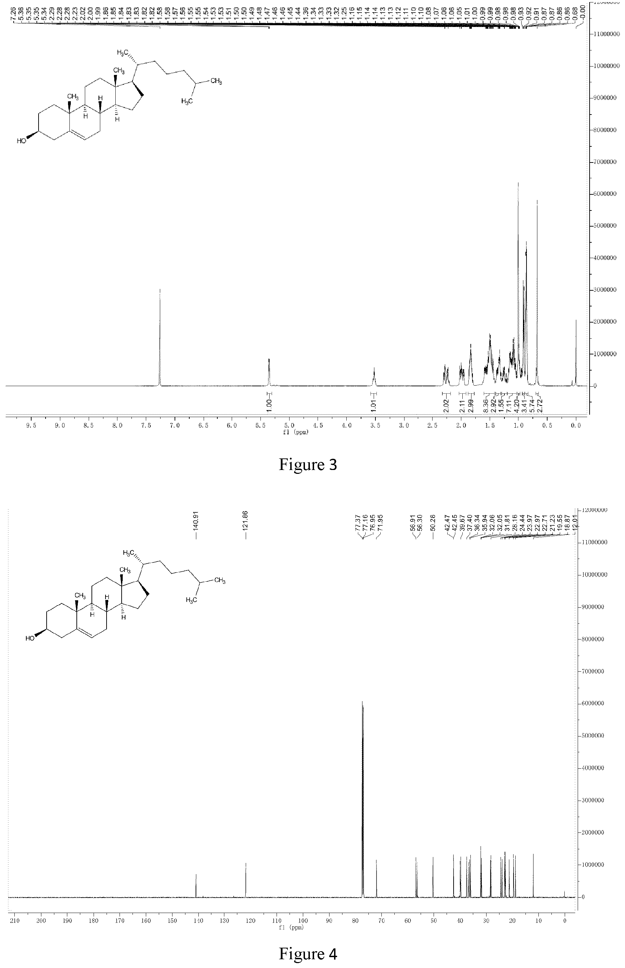

Figure 3 shows the ¹H NMR spectrum (CDCl₃ ) of cholesterol.
Figure 4 shows the ¹³C NMR spectrum (CDCl₃ ) of cholesterol.
Figure 5 shows the gas chromatogram of the refined compound of formula (7-1) obtained in Example 7 of the present invention.
Figure 6 shows a gas chromatogram of cholesterol obtained by hydrolysis of the compound of formula (7-1) in Example 8 of the present invention.

### PREFERRED EMBODIMENTS OF THE INVENTION

The following examples are given to further illustrate the specific solutions of the present invention. The process, conditions, reagents and experimental methods of the implementation of the present invention are all general knowledge and common knowledge in the field except for the contents specially mentioned below, and the present invention has no special limitation.

In the following examples, the structure of the compound is determined by nuclear magnetic resonance instrument and high-resolution mass spectrometer; reagents are mainly provided by Shanghai Sinopharm Chemical Reagent Company; product purification is mainly through slurry and column chromatography; silica gel (200-300) is produced by Qingdao Ocean Chemical Factory.

The present invention provides a method for synthesizing cholesterol by taking plant-derived 21-hydroxyl-20-methylpregn-4-en-3-one BA as a raw material, and its reaction process is shown in the aforementioned route (A), it should be noted that the present invention also includes the generation of other by-products in the method of synthesizing cholesterol by taking plant-based 21-hydroxyl-20-methylpregn-4-en-3-one BA as the raw material, the specific reaction route is shown in (A') :

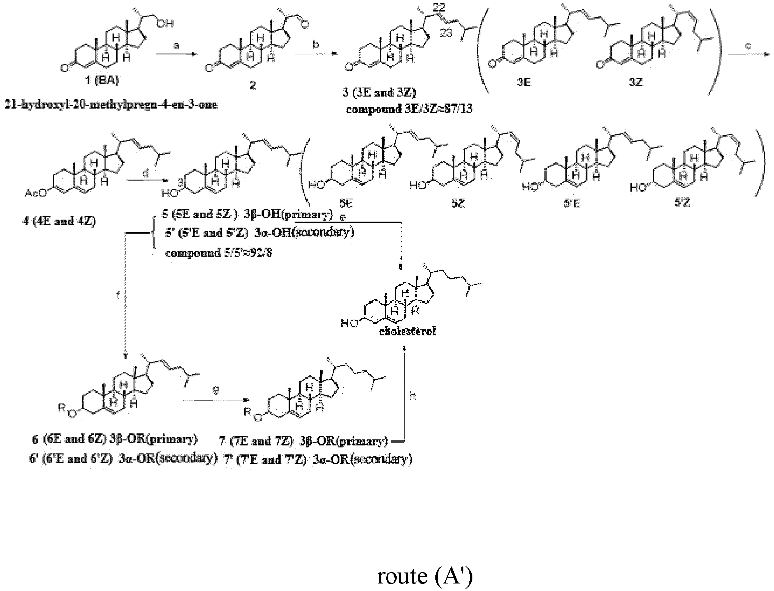

Note: In the compounds of formula (3) to (6), the double bonds between C-22 and C-23 are dominated by the E configuration, supplemented by the Z configuration, and the ratio of the two configurations is E/Z≈87/13 (¹H NMR judgment), if there is no subsequent purification operation, the ratio remains unchanged; the ratio of the compounds of formula (5)(3β-OH / (5')(3α-OH) is about 92/8 (¹H NMR judgment), if there is no subsequent purification operation, the ratio of the compounds of formula (6)/(6'), (7)/(7') remains unchanged.

The compounds of formula (3), formula (4), formula (5), formula (5'), formula (6) and formula (6') of the present invention have cis-trans configuration (E and Z configuration) due to the D ring side chain double bond (C-22, C-23 double bond) introduced by Wittig reaction, with a ratio of the two configurations is E/Z≈87/13 (¹H NMR judgment), if there is no subsequent purification operation, the ratio remains unchanged; the compounds of formula (5) (3β-OH) and (5') (3α-OH) are obtained by reduction of the compound of formula (4) through NaBH₄ or KBH₄, and the ratio of 3β-OH/3α-OH in the compounds of formula(5) (3β-OH) and (5') (3α-OH) is about 92/8 (¹H NMR judgment), if there is no subsequent purification operation, the ratio remains unchanged. Therefore the compounds of formula (3), formula (4), formula (5), formula (5'), formula (6), formula (6'), formula (7) and formula (7') are not single substances, but mixtures.

### Example 1 The preparation of the compound of formula (2)

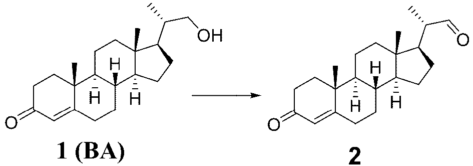

The present example provides the preparation result of the compound of formula (2) under different experimental conditions:
(1) BA (50.00g, 0.15mol), TEMPO (235mg, 1.50mmol), dichloromethane (400mL), sodium bicarbonate (17.60g, 0.21mol), NCS (23.10g, 173.00mmol), tetrabutylammonium bromide (4.84g, 15mmol) and water (160mL) were added in sequence to a flask and reacted for 6h at 0°C. After the completion of the reaction was detected by TLC, sodium thiosulfate pentahydrate solution (11.25g sodium thiosulfate pentahydrate/250mL water) was added, stirred for 30min at 5-10°C, separated, and the aqueous phase was extracted with dichloromethane (300mL×2). The organic layers were combined, washed with 1% sodium hydroxide solution (300mL), separated, the organic phase was dried with anhydrous sodium sulfate, and concentrated under reduced pressure to afford the compound of formula (2) (47.46g, white solid, molar yield of 94.9%). ¹H NMR (400 MHz, CDCl₃) *δ* 9.55 (s, 1H), 5.71 (s, 1H), 2.45 - 2.23 (m, 5H), 1.99 (t, *J =* 13.7 Hz, 2H), 1.91 - 1.78 (m, 2H), 1.68 (t, *J =* 10.2 Hz, 2H), 1.43 (m, 5H), 1.30 - 1.19 (m, 2H), 1.17 (s, 3H), 1.11 (d, *J =* 5.5 Hz, 3H), 1.06 - 0.89 (m, 3H), 0.75 (s, 3H). ¹³C NMR (100 MHz, CDCl₃) *δ* 205.00, 199.65, 171.31, 123.99, 55.25, 53.84, 51.04, 49.54, 43.10, 39.39, 38.68, 35.80, 35.68, 34.06, 32.93, 32.05, 27.11, 24.64, 21.06, 17.48, 13.53, 12.44. HRMS(ESI): calcd for C₂₂H₃₂NaO₂ [M+Na]⁺, 351.2295, found 351.2292.
(2) BA (50g, 0.15mol), TEMPO (236mg, 1.5mmol), dichloromethane (400mL), sodium bicarbonate (17.6g, 0.21mol), NBS (35.6g, 200mmol), tetrabutylammonium bromide (4.84g, 15mmol) and water (320mL) were added in sequence to a flask and reacted for 6h at 0°C. After the completion of the reaction was detected by TLC, sodium thiosulfate pentahydrate solution (16g sodium thiosulfate pentahydrate/300mL water) was added, stirred for 20min at 5-10°C, separated, and the aqueous phase was extracted with dichloromethane (300mL×2). The organic layers were combined, washed with 1% sodium hydroxide solution (200 mL), separated, the organic phase was dried with anhydrous sodium sulfate, and concentrated under reduced pressure to afford the compound of formula (2) (47.5 g of white solid, molar yield of 95%).
(3) The compound of formula (1) BA (50g, 0.15mol), IBX (84g, 0.3mmol), THF (350mL) and DMSO (300mL) were added in sequence to a flask, and reacted for 5h at 25°C. After the completion of the reaction was detected by TLC, water was added, suction filtered, and the filtrate was concentrated under reduced pressure, added with dichloromethane (500mL) and water (300mL) for extraction. The organic phase was washed with 1% sodium hydroxide solution (300mL), dried with anhydrous sodium sulfate, concentrated under reduced pressure to afford the compound of formula (2) (46.7 g of white solid, molar yield of 93.4%).

### Example 2 The preparation of the compound of formula (3)

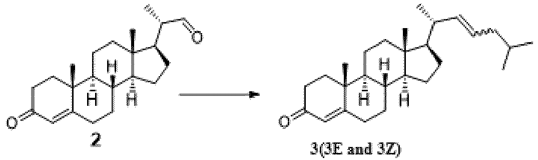

The present example provides the preparation result of the compound of formula (3) under different experimental conditions:
(1) Triphenylphosphine (5.25g, 20mmol), 1-bromo-3-methylbutane (6.04g, 40mmol), and 25mL of toluene were added to a flask, heated and reacted under reflux for 72h, cooled to 25°C, suction filtered to afford a quaternary phosphonium salt (5.12g of white solid , molar yield of 62%).

Under the ice bath, potassium tert-butoxide (1.39g, 12.4mmol) was added to the above-mentioned quaternary phosphonium salt (5.12g, 12.4mmol) in toluene (25ml), stirred for 0.5h, then added with the compound of formula (2) (2.63g, 8mmol) in batches, reacted for 0.5h at 10°C. After the completion of the reaction was monitored by TLC, water (5 mL) was added to quench the reaction, 2M HCl was added to adjust the pH to 6-7, the organic phase was washed with water (30 mL×2), and concentrated under reduced pressure to afford a milky white solid. The above-mentioned solid was added to a mixed solution of ethanol/water, slurried for 1 h at 25°C, and filtered to afford the compound of formula (3) (3E and 3Z, 3E/3Z≈87/13, 2.76 g of white solid, molar yield of 90.2%). HRMS(ESI): calcd for C₂₇H₄₂NaO [M+Na]⁺, 405.3128, found 405.3136.

Note: In the present invention, the ratio of the E/Z configuration of the intermediate compound of formula (4), the compound of formula (5) and the compound of formula (6) obtained through acetylation and reduction of the compound of formula (3) remains basically unchanged, and the double bond between C-22 and C-23 is dominated by E configuration, supplemented by Z configuration (3E/3Z≈87/13). Since the cis-trans isomers of the double bond between the C-22 and C-23 positions in the compound of formula (6) are subjected to Raney Ni hydrogenation reduction, deprotection or hydrolysis to afford the product cholesterol, therefore, the ratio of the E/Z configuration of the corresponding compound is no longer marked in the following examples.

(2) Triphenylphosphine (5.25g, 20mmol), 1-bromo-3-methylbutane (4.53g, 30mmol), 25mL of xylene were added to a flask, heated and reacted under reflux for 48h, then cooled to 25°C, suction filtered to afford a quaternary phosphonium salt (7.76 g of white solid, molar yield of 93.94%).

In the ice bath, potassium tert-butoxide (2.11g, 18.79mmol) was added to the above-mentioned quaternary phosphonium salt (7.76g, 18.79mmol) in xylene (25mL), stirred for 0.5h, then added with the compound of formula (2) (4.92g, 15mmol) in batches, reacted for 1h at 5°C. After the completion of the reaction was monitored by TLC, water (5 mL) was added to quench the reaction, 2M HCl was added to adjust the pH to 6-7, the organic phase was washed with water (40 mL×2), and concentrated under reduced pressure to afford a milky white solid. The above-mentioned solid was added to a mixed solution of ethanol/water, slurried for 1 h at 25°C, and filtered to afford the compounds of formula (3) (3E and 3Z, 5.38 g of white solid, molar yield of 93.9%).

(3) Triphenylphosphine (5.25g, 20mmol), 1-chloro-3-methylbutane (3.2g, 30mmol), xylene 25mL were added to a flask, heated and reacted under reflux for 72h, then cooled to 25°C, suction filtered to afford a quaternary phosphonium salt (6.12g of white solid, molar yield of 83.04%).

In the ice bath, potassium tert-butoxide (1.86 g, 16.59 mmol) was added to the above-mentioned quaternary phosphonium salt (6.12 g, 16.59 mmol) in xylene (25 mL), stirred for 0.5 h, then added with the compound of formula (2) in batches (4.58g, 12mmol), and reacted for 0.5h at 10°C. After the completion of the reaction was monitored by TLC, water (5 mL) was added to quench the reaction, 2M HCl was added to adjust the pH to 6-7, the organic phase was washed with water (30 mL×2), and concentrated under reduced pressure to afford a milky white solid. The above-mentioned solid was added to a mixed solution of ethanol/water, slurried for 1 h at 25°C, and filtered to afford the compound of formula (3) (3E and 3Z, 4.04 g of white solid, molar yield of 88.07%).

### Example 3 The preparation of the compound of formula (4)

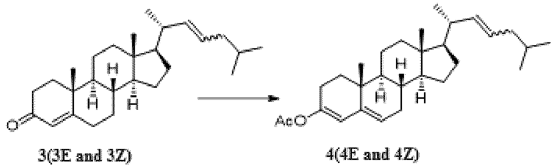

The present example provides the preparation result of the compound of formula (4) under different experimental conditions:
(1) The compound of formula (3) (9.94g, 26mmol), acetic anhydride (63.71g, 624mmol), acetyl chloride (51.03g, 650mmol) and diisopropylamine (10.5g, 103.8mmol) were added to a flask, reacted under reflux for 5h, after the completion of the reaction was monitored by TLC, cooled to 25°C, concentrated under reduced pressure, cooled slightly. The reaction solution was added to ice water (150mL) and stirred for 15min, filtered, drained, and the resulting solid was added to methanol (20mL) and pyridine (1 mL), slurried for 30 min at 25°C, filtered. The filter cake was rinsed with methanol (4 mL), suction filtered, and dried to afford the compound of formula (4) (4E and 4Z, 10.47 g of white solid, molar yield of 94.97%).
(2) The compound of formula (3) (9.94g, 26mmol), acetic anhydride (39.46g, 390mmol), acetyl chloride (8.16g, 104mmol) and DIPEA (13.39g, 103.6mmol) were added to a flask, reacted under reflux for 4h, after the completion of the reaction was monitored by TLC, cooled to 25°C, concentrated under reduced pressure, cooled slightly. The reaction solution was added to ice water (150mL) and stirred for 15min, filtered, drained, and the resulting solid was added to methanol (20mL) and pyridine (1mL) , slurried for 30 min at 25°C, filtered. The filter cake was rinsed with methanol (4 mL), suction filtered, and dried to afford the compound of formula (4) (4E and 4Z, 10.15 g of white solid, molar yield of 92.07%).
(3) The compound of formula (3) (9.94g, 26mmol), acetic anhydride (63.71g, 624mmol), acetyl chloride (51.03g, 650mmol) were added to a flask, reacted under reflux 4h, after the completion of the reaction was monitored by TLC, cooled to 25 °C, concentrated under reduced pressure, cooled slightly. The reaction solution was added to ice water (150mL) and stirred for 15min, filtered, drained, and the resulting solid was added to methanol (20mL) and pyridine (1mL), slurried for 30 min at 25°C, filtered. The filter cake was rinsed with methanol (4 mL), suction filtered, and dried to afford the compound of formula (4) (4E and 4Z, 9.8 g of white solid, molar yield of 88.9%).

### Example 4 The preparation of the crude compound of formula (5)

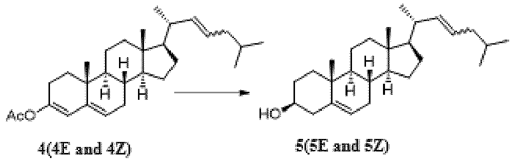

The present example provides the preparation result of the crude compound of formula (5) under different experimental conditions:
(1) The compound of formula (4) (8.48g, 20mmol), the mixed solvent (170mL, V/V/V=10/5/3) of tetrahydrofuran, ethanol and water were added to a flask, added with sodium borohydride (3.03g, 80mmol) in batches, and reacted for 8h at 30°C. After the completion of the reaction was monitored by TLC, 6M hydrochloric acid was added to adjust the reaction solution pH=7-8, and a large amount of solids were precipitated in the reaction solution, filtered. The filtrate was taken, evaporated under reduced pressure to remove the solvent, and a large amount of solids were precipitated, added with dichloromethane (100mL) and water (50mL) for extraction, separated. The organic phase was washed with water (100mL), stirred, layered, and evaporated under reduced pressure to remove the solvent to afford the crude compound of formula (5) (7.53g of light yellow solid, molar yield of 98.04%).
   Note: The main components of the crude compound of formula (5) obtained with sodium borohydride as the reducing agent are the compounds (5) (5E and 5Z, 3β-OP) and (5') (5'E and 5'Z, 3α-OH ), according to ¹H NMR judgement, the ratio is the compounds (5)/(5')≈92/8. The ratio of 5(3β-OH)/5'(3α-OH) in the crude compounds of formula (5) obtained in the following examples is basically the same as that in this example, and will not be noted later.
(2) The compound of formula (4) (8.48g, 20mmol), the mixed solvent (95mL, V/V/V=18/9/1) of dichloromethane, methanol and water were added to a flask, added with sodium borohydride(1.51g, 40mmol) in batches, and reacted for 6h at 25°C. After the completion of the reaction was monitored by TLC, 3M hydrochloric acid was added to adjust the reaction solution pH=7-8, and a large amount of solids were precipitated in the reaction solution, filtered. The filtrate was taken, evaporated under reduced pressure to remove the solvent, and a large amount of solids were precipitated, added with dichloromethane (100mL) and water (50mL) for extraction, separated. The organic phase was washed with water (100mL), stirred, layered, and evaporated under reduced pressure to remove the solvent to afford the crude compound of formula (5) (7.5g of light yellow solid, molar yield of 97.9%).
(3) The compound of formula (4) (8.48g, 20mmol), the mixed solvent (200mL, V/V/V=10/5/3) of tetrahydrofuran, ethanol and water were added to a flask, added with potassium borohydride ( 4.86g, 90mmol) in batches, and reacted for 8h at 30°C. After the completion of the reaction was monitored by TLC, 6M hydrochloric acid was added to adjust the reaction solution pH=7-8, a large amount of solids were precipitated in the reaction solution, filtered. And the filtrate was taken, evaporated under reduced pressure to remove the solvent, and a large amount of solids were precipitated, added with dichloromethane (100mL) and water (50mL) for extraction, separated. The organic phase was washed with water (100mL), stirred, layered, and evaporated under reduced pressure to remove the solvent to afford the crude compound of formula (5) (7.57g of light yellow solid, molar yield of 98.6%).

Note: The main components of the crude compound of formula (5) obtained by reduction of potassium borohydride are the compounds (5) (5E and 5Z, 3β-OH) and (5') (5'E and 5'Z, 3α-OH), among them, the content of the compound 5'(3α-OH) is relatively high, the compounds 5(3β-OH)/5'(3α-OH)≈85/15 according to ¹H NMR judgement.

### Example 5 Preparation of cholesterol by selective hydrogenation reduction of the compound of formula (5)

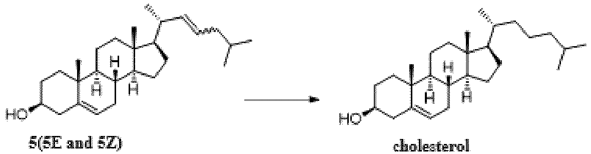

The above-mentioned crude compound of formula (5) was purified by column chromatography (petroleum ether/ethyl acetate=40/1, v/v) to afford the compounds of formula (5) (5E and 5Z).

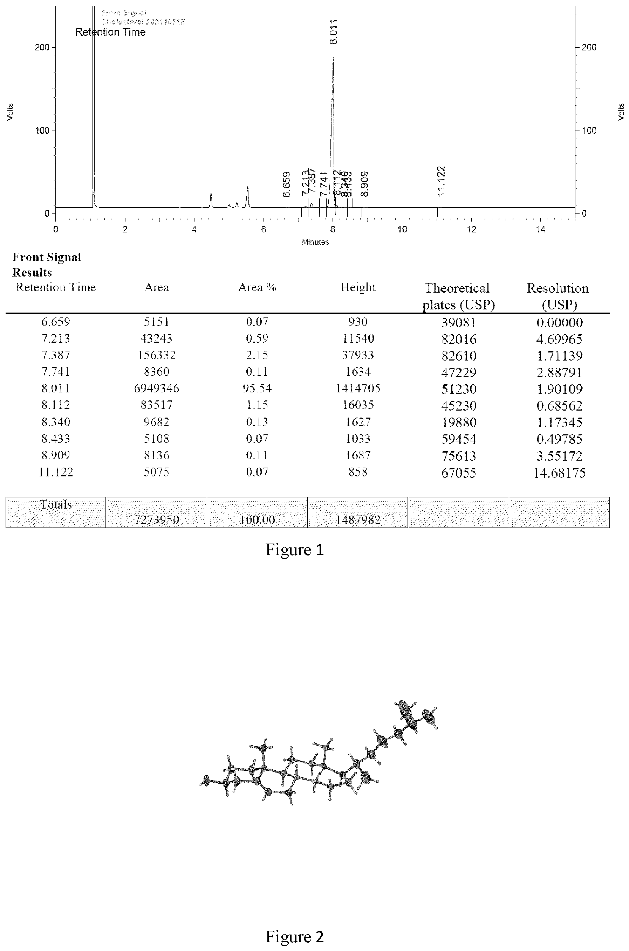

The purified compound of formula (5) (5E and 5Z, 3.84g, 10mmol), Raney Ni (3.9g, wet weight), isopropanol (40mL), H₂ (1atm) were added to a flask, and reacted for 6h at 30°C, after the completion of the reaction was monitored by TLC, filtered to remove Raney Ni. The filtrate was concentrated under reduced pressure to afford crude product cholesterol, and subjected to recrystallization purification (ethanol/water) to afford cholesterol (3.8g of white solid , molar yield of 98.9%, gas chromatography purity of 95.54% , see Figure 1, CCDC 2099442, see Figure 2 for the single crystal structure). mp: 147-149°C_{∘} ¹H NMR (600 MHz, CDCl₃) *δ* 5.36 - 5.34 (m, 1H), 3.55 - 3.49 (m, 1H), 2.35 - 2.19 (m, 2H), 2.06 - 1.92 (m, 2H), 1.86 - 1.79 (m, 3H), 1.68 - 1.19 (m, 14H), 1.19 - 1.03 (m, 7H), 1.01 (s, 3H), 0.98 - 0.93 (m, 1H), 0.91 (d, *J =* 6.6 Hz, 3H), 0.86 (dd, *J =* 6.6, 2.8 Hz, 6H), 0.68 (s, 3H).¹³C NMR (150 MHz, CDCl₃) *δ* 140.77, 121.73, 71.82, 56.78, 56.17, 50.14, 42.33, 42.32, 39.80, 39.53, 37.27, 36.52, 36.20, 35.80, 31.93, 31.92, 31.68, 28.25, 28.03, 24.31, 23.84, 22.84, 22.58, 21.10, 19.41, 18.73, 11.87.HRMS(ESI): calcd for C₂₇H₄₆NaO [M+Na]⁺,409.3441, found 409.3121.

### Example 6 The preparation of the crude compound of formula (6)

### 1. Preparation of the crude compound of formula (6-1)

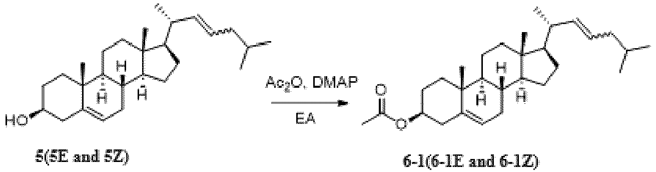

Ethyl acetate (76mL), the crude compound of formula (5) (7.68g, 20mmol) were added to a flask, stirred to dissolve and be clarified, added with DMAP (0.245g, 2mmol) and acetic anhydride (6.13g, 60mmol), and reacted for 4h at 45°C, after the completion of the reaction was monitored by TLC, added with water (20mL) to quench the reaction, added with ethyl acetate (100mL) for extraction. The organic phase was washed with water, washed with saturated brine, and concentrated under reduced pressure to afford the crude compound of formula (6-1) (8.18g of white solid, molar yield of 95.9%).

Note: The main components of the crude compound of formula (6-1) are the compounds (6-1) (6-1E and 6-1Z) and (6'-1) (6'-1E and 6'-1Z), and the ratio is the compounds (6-1)/(6'-1)≈92/8.

### 2. Preparation of the crude compound of formula (6-2)

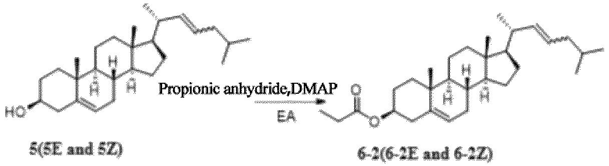

Ethyl acetate (50mL), the crude compound of formula (5) (7.68g, 20mmol) were added to a flask, stirred to dissolve and be clarified, added with DMAP (0.245g, 2mmol) and propionic anhydride (5.3g, 40mmol), and reacted for 5h at 50°C, after the completion of the reaction was monitored by TLC, added with water (20mL) to quench the reaction, added with ethyl acetate (100mL) for extraction. The organic phase was washed with water, washed with saturated brine, dried with anhydrous Na₂SO₄, concentrated under reduced pressure, and added with methanol/water, slurried for 5 hours at 25°C, suction filtered to afford the crude compound of formula (6-2) (8.64g of white solid, molar yield of 98.07%).

Note: The main components of the crude compound of formula (6-2) are the compounds (6-2) (6-2E and 6-2Z) and (6'-2) (6'-2E and 6'-2Z), and the ratio is the compounds (6-2)/(6'-2)≈92/8.

### 3. Preparation of the crude compound of formula (6-3)

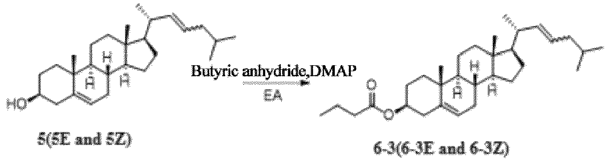

Ethyl acetate (50mL), the crude compound of formula (5) (7.68g, 20mmol) were added to a flask, stirred to dissolve and be clarified, added with DMAP (0.245g, 2mmol) and butyric anhydride (9.54g, 60mmol), and reacted for 4h at 55°C, after the completion of the reaction was monitored by TLC, added with water (20mL) to quench the reaction, added with ethyl acetate (100mL) for extraction. The organic phase was washed with water, washed with saturated brine, dried with anhydrous Na₂SO₄, concentrated under reduced pressure, and added with methanol/water, slurried for 5 hours at 25°C, suction filtered to afford the crude compound of formula (6-3) (8.82g of white solid, molar yield of 97.03%).

Note: The main components of the crude compound of formula (6-3) are the compounds (6-3) (6-3E and 6-3Z) and (6'-3) (6'-3E and 6'-3Z), and the ratio is the compounds (6-3)/(6'-3)≈92/8.

### 4. Preparation of the crude compound of formula (6-4)

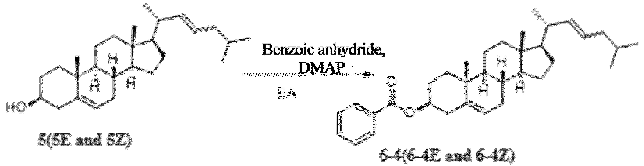

Ethyl acetate (115mL), the crude compound of formula (5) (7.68g, 20mmol) were added to a flask, stirred to dissolve and be clarified, added with DMAP (0.245g, 2mmol) and benzoic anhydride (13.5g, 60mmol), and reacted for 4 hours at 45°C, after the completion of the reaction was monitored by TLC, concentrated under reduced pressure, added with methanol/water, slurried for 5 hours at 25°C, and suction filtered to afford the crude compound of formula (6-4) (9.38g of white solid, molar yield of 95.9%).

Note: The main components of the crude compound of formula (6-4) are the compounds (6-4) (6-4E and 6-4Z) and (6'-4) (6'-4E and 6'-4Z), and the ratio is the compounds (6-4)/(6'-4)≈92/8.

### 5. Preparation of the crude compound of formula (6-5)

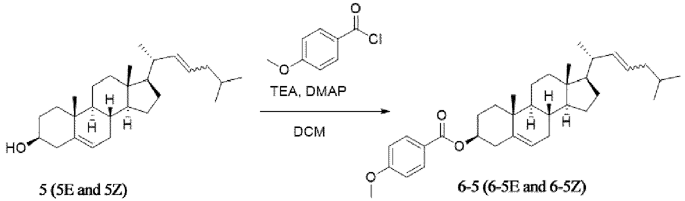

DCM (50mL), the crude compound of formula (5) (7.68g, 20mmol) were added to a flask, stirred to dissolve and be clarified, added with triethylamine (2.64g, 26.14mmol) and DMAP (0.245g, 2mmol), and added dropwise slowly with p-chlorobenzoyl chloride (6.8g, 39.2mmol) at 0°C, protected by N₂, and reacted for 12h at 35°C, after the completion of the reaction was monitored by TLC, added with water (30mL) to quench the reaction, extracted with DCM (80mL). The organic phase was washed with saturated NaHCO₃ aqueous solution, 2N diluted hydrochloric acid, water, saturated NaCl in sequence, concentrated under reduced pressure, added with methanol/water, slurried for 5 hours at 25°C, and suction filtered to afford the crude compound of formula (6-5) (9.86g of white solid, molar yield of 95.04% ).

Note: The main components of the crude compound of formula (6-5) are the compounds (6-5) (6-5E and 6-5Z) and (6'-5) (6'-5E and 6'-5Z), and the ratio is the compounds (6-5)/(6'-5)≈92/8.

### 6. Preparation of the crude compound of formula (6-6)

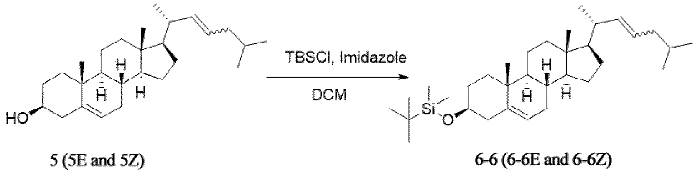

DCM (100mL), the crude compound of formula (5) (7.68g, 20mmol) were added to a flask, stirred to dissolve and be clarified, added with TBSCl (9.85g, 65.35mmol) and imidazole (7.12g, 104.56mmol), and reacted for 12h at 25°C, after the completion of the reaction was monitored by TLC, added with water (100mL) and stirred for 10min, extracted with DCM (80mL), separated. The organic phase was washed with water and saturated NaCl, concentrated under reduced pressure, added with methanol/water, slurried for 3 hours at 25°C, and suction filtered to afford the crude compound of formula (6-6) (9.58g of white solid, molar yield of 96.09%).

Note: The main components of the crude compound of formula (6-6) are the compounds (6-6) (6-6E and 6-6Z) and (6'-6) (6'-6E and 6'-6Z), and the ratio is the compounds (6-6)/(6'-6)≈92/8.

### Example 7 The preparation of the compound of formula (7)

### 1. The preparation of the compound of formula (7-1)

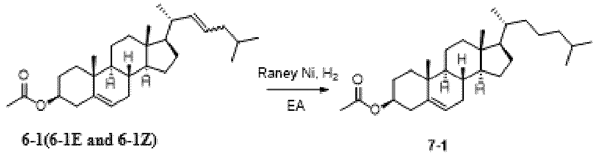

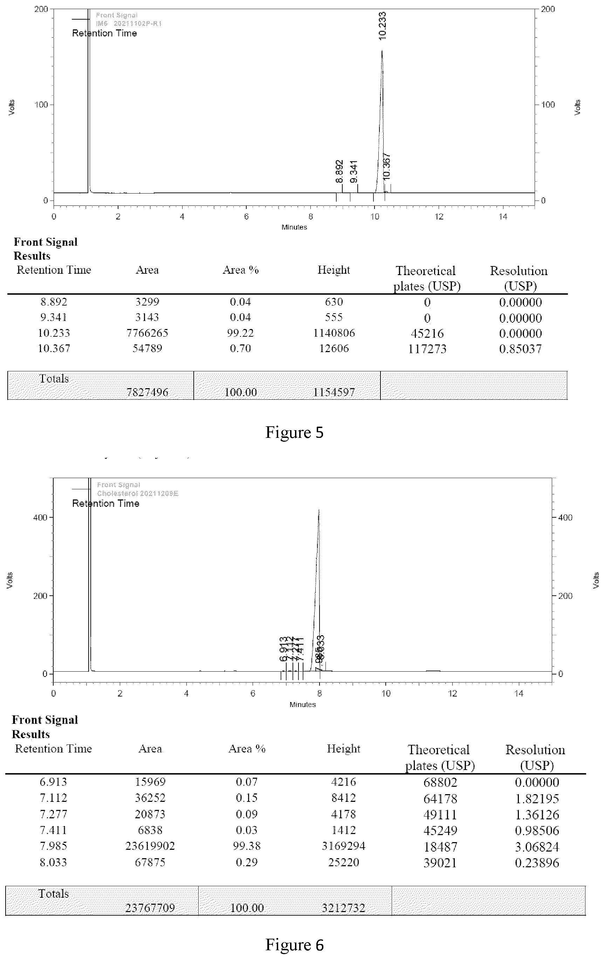

Ethyl acetate (75mL), the crude compound of formula (6-1) (10g, 23.54mmol) were added to a flask, stirred to dissolve and be clarified, then added with Raney Ni (10g, wet weight, activated according to the standard procedure), H₂ (1atm), and reacted for 7h at 35°C, after the completion of the reaction was monitored by gas phase, filtered to remove Raney Ni through diatomaceous earth. The filtrate was concentrated under reduced pressure to afford the crude compound of formula (7-1), which was directly used in the purification step. The white solid was added to a mixed solution of methanol and ethyl acetate, heated reflux until dissolved and clarified, cooled naturally to 15°C, stirred for 4h, and suction filtered to afford the compound of formula (7-1) (8.4g of white solid, molar yield of 84.1 %, gas chromatography purity of 99.22%, Figure 5). ¹H NMR (600 MHz, CDCl₃) δ 5.40 (d, *J =* 4.9 Hz, 1H), 4.68 - 4.57 (m, 1H), 2.40 - 2.31 (m, 2H), 2.05 (s, 3H), 2.05 - 1.95 (m, 2H), 1.91 - 1.81 (m, 3H), 1.66 - 1.43 (m, 8H), 1.41 - 1.33 (m, 3H), 1.31 - 1.24 (m, 1H), 1.22 - 1.07 (m, 7H), 1.04 (s, 3H), 1.03 - 0.96 (m, 2H), 0.94 (d, *J =* 6.5 Hz, 3H), 0.89 (dd, *J =* 6.6, 2.8 Hz, 6H), 0.70 (s, 3H).¹³C NMR (151 MHz, CDCl₃) δ 170.55, 139.67, 122.66, 73.99, 56.69, 56.14, 50.04, 42.32, 39.74, 39.53, 38.13, 37.00, 36.60, 36.19, 35.80, 31.91, 31.87, 28.24, 28.02, 27.78, 24.29, 23.83, 22.83, 22.57, 21.45, 21.03, 19.32, 18.72, 11.86.

Note: The main components of the crude compound of formula (7-1) are the compounds (7-1) and (7'-1), and the ratio is the compounds (7-1)/(7'-1)≈92/8.

### 2. Preparation of the compound of formula (7-2)

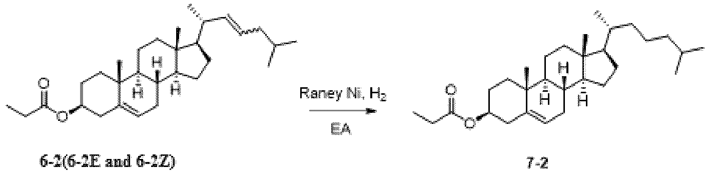

Ethyl acetate (75mL), the crude compound of formula (6-2) (10.05g, 22.8mmol) were added to a flask, stirred to dissolve and be clarified, then added with Raney Ni (15g, wet weight, activated according to the standard procedure), H₂ (1atm ), and reacted for 7 h at 55°C, after the completion of the reaction was monitored by gas phase, filtered to remove Raney Ni through diatomaceous earth. The filtrate was concentrated under reduced pressure to afford the crude compound of formula (7-2) as a white solid, which was directly used in the purification step. The white solid was added to a mixed solution of methanol and ethyl acetate, heated reflux until dissolved and clarified, cooled naturally to 15°C, stirred for 4h, and suction filtered to afford the compound of formula (7-2) (8.17g of white solid, molar yield of 81%, gas chromatographic purity of 98.69%). ¹H NMR (600 MHz, CDCl₃) δ 5.39 (d, *J =* 4.9 Hz, 1H), 4.67 - 4.62 (m, 1H), 2.44 - 2.24 (m, 4H), 2.10 - 1.95 (m, 2H), 1.93 - 1.81 (m, 3H), 1.68 - 1.43 (m, 8H), 1.40 - 1.34 (m, 3H), 1.29-1.25 (m, 1H), 1.20-1.10 (m, 9H), 1.04 (s, 3H), 1.03 - 0.96 (m, 3H), 0.94 (d, *J =* 6.5 Hz, 3H), 0.89 (dd, *J =* 6.6, 2.8 Hz, 6H), 0.70 (s, 3H). ¹³C NMR (151 MHz, CDCl₃) δ 173.96, 139.75, 122.59, 56.70, 56.14, 50.03, 42.32, 39.74, 39.53, 38.16, 37.01, 36.61, 36.19, 35.80, 31.91, 31.87, 28.24, 28.02, 27.94, 27.81, 24.29, 23.83, 22.83, 22.57, 21.04, 19.33, 18.72, 11.86, 9.19.

Note: The main components of the crude compound of formula (7-2) are the compounds (7-2) and (7'-2), and the ratio is the compounds (7-2)/(7'-2)≈92/8.

### 3. Preparation of the compound of formula (7-3)

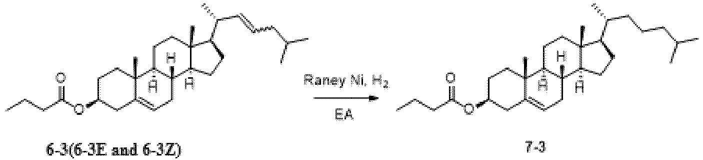

Ethyl acetate (75mL), the crude compound of formula (6-3) (10g, 22mmol) were added to a flask, stirred to dissolve and be clarified, then added with Raney Ni (10g, wet weight, activated according to the standard procedure), H₂ (1atm), and reacted for 10 h at 35°C, after the completion of the reaction was monitored by gas phase, filtered to remove Raney Ni through diatomaceous earth. The filtrate was concentrated under reduced pressure to afford the crude compound of formula (7-3) as a white solid, which was directly used in the purification step. The white solid was added to a mixed solution of methanol and ethyl acetate, heated reflux until dissolved and clarified, cooled naturally to 15°C, stirred for 5h, and suction filtered to afford the compound of formula (7-3) (8.3g of white solid, molar yield of 82.5%, gas chromatographic purity of 99.1%). ¹H NMR (600 MHz, CDCl₃) δ 5.39 (d, *J =* 4.9 Hz, 1H), 4.74 - 4.58 (m, 1H), 2.33 (d, *J =* 7.1 Hz, 2H), 2.28 (t, *J =* 7.4 Hz, 2H), 2.09 - 1.95 (m, 2H), 1.93 - 1.80 (m, 3H), 1.68 - 1.65 (m, 2H), 1.65 - 1.41 (m, 7H), 1.41 - 1.31 (m, 3H), 1.31 - 1.24 (m, 1H), 1.23 - 1.06 (m, 7H), 1.04 (s, 3H), 1.03-0.99 (m, 2H), 0.99 - 0.92 (m, 7H), 0.89 (dd, *J* = 6.7, 2.7 Hz, 6H), 0.70 (s, 3H). ¹³C NMR (151 MHz, CDCl₃) δ 173.14, 139.74, 122.59, 73.68, 56.70, 56.14, 50.03, 42.32, 39.74, 39.53, 38.18, 37.01, 36.60, 36.19, 35.80, 31.91, 31.87, 28.24, 28.02, 27.83, 24.29, 23.84, 22.83, 22.57, 21.04, 19.33, 18.72, 18.56, 13.65, 11.86.

Note: The main components of the crude compound of formula (7-3) are the compounds (7-3) and (7'-3), and the ratio is the compounds (7-3)/(7'-3)≈92/8.

### 4. Preparation of the compound of formula (7-4)

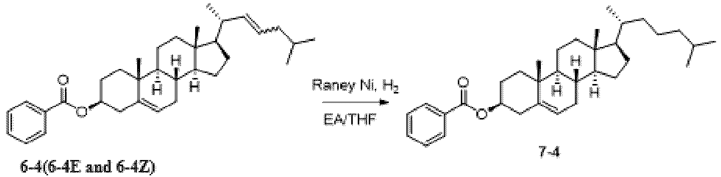

Tetrahydrofuran (35mL), ethyl acetate (35mL), the crude compound of formula (6-4) (10g, 20.46mmol) were added to a flask, stirred to dissolve and be clarified, then added with Raney Ni (10g, wet weight, activated according to the standard procedure), H₂ (1 atm), and reacted for 24 hours at 35°C, after the completion of the reaction was monitored by gas phase, filtered to remove Raney Ni through diatomaceous earth. The filtrate was concentrated under reduced pressure to afford the crude compound of formula (7-4) as a white solid, which was directly used in the purification step. The white solid was added to a mixed solution of methanol and ethyl acetate, heated reflux until dissolved and clarified, cooled naturally to 15°C, stirred for 4-5h, and suction filtered to afford the compound of formula (7-4) (8.33g of white solid , molar yield of 82.96%, gas chromatography purity of 98.96%). ¹H NMR (600 MHz, CDCl₃) δ 8.11 - 7.99 (m, 2H), 7.59 - 7.51 (m, 1H), 7.43 (t, *J =* 7.8 Hz, 2H), 5.42 (dd, *J =* 5.1, 2.1 Hz, 1H), 4.89 - 4.84 (m, 1H), 2.47 (d, *J =* 8.0 Hz, 2H), 2.05-1.97 (m, 3H), 1.94 - 1.90 (m, 1H), 1.86-1.80 (m, 1H), 1.77 - 1.73 (m, 1H), 1.63 - 1.45 (m, 6H), 1.41 - 1.30 (m, 3H), 1.29 - 1.05 (m, 11H), 1.04 - 0.99 (m, 3H), 0.92 (d, *J =* 6.5 Hz, 3H), 0.87 (dd, *J* = 6.6, 2.8 Hz, 6H), 0.69 (s, 3H). ¹³C NMR (151 MHz, CDCl₃) δ 166.01, 139.69, 132.72, 130.87, 129.55, 128.27, 122.80, 56.71, 56.15, 50.06, 42.34, 39.76, 39.54, 38.23, 37.05, 36.68, 36.20, 35.82, 31.95, 31.90, 28.25, 28.03, 27.90, 24.31, 23.85, 22.84, 22.58, 21.07, 19.40, 18.74, 11.88.

Note: The main components of the crude compound of formula (7-4) are the compounds (7-4) and (7'-4), and the ratio is the compounds (7-4)/(7'-4)≈92/8.

### 5. Preparation of the compound of formula (7-5)

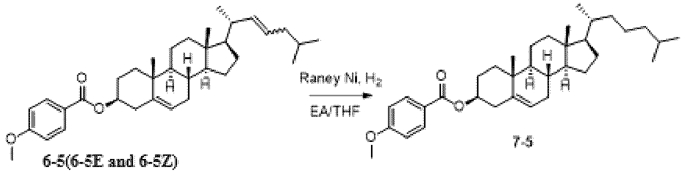

Tetrahydrofuran (39mL) and ethyl acetate (39mL), the crude compound of formula (6-5) (10g, 19.2mmol) were added to a flask, stirred to dissolve and be clarified, then added with Raney Ni (15g, wet weight, activated according to the standard procedure), H₂ (1 atm), and reacted for 20 h at 30°C, after the completion of the reaction was monitored by gas phase, filtered to remove Raney Ni through diatomaceous earth. The filtrate was concentrated under reduced pressure to afford the crude compound of formula (7-5) as a white solid, which was directly used in the purification step. The white solid was added to a mixed solution of methanol and ethyl acetate, heated reflux until dissolved and clarified, naturally cooled to 15°C, stirred for 4h, and suction filtered to afford the compound of formula (7-5) (8.1g of white solid, molar yield of 81%, gas chromatographic purity of 98.5%). ¹H NMR (500 MHz, CDCl₃) δ 8.02 (d, *J* = 8.7 Hz, 2H), 6.93 (d, *J =* 8.7 Hz, 2H), 5.44 (d, *J =* 5.0 Hz, 1H), 4.88 - 4.82 (m, 1H), 3.88 (s, 3H), 2.47 (d, *J =* 7.9 Hz, 2H), 2.06 - 1.98 (m, 3H), 1.95 - 1.91 (m, 1H), 1.90 - 1.81 (m, 1H), 1.79 - 1.69 (m, 1H), 1.67 - 1.44 (m, 7H), 1.36 (d, *J =* 8.9 Hz, 3H), 1.30 - 1.10 (m, 7H), 1.09 (s, 3H), 1.07 - 0.99 (m, 3H), 0.94 (d, *J =* 6.5 Hz, 3H), 0.89 (dd, *J =* 6.6, 2.4 Hz, 6H), 0.71 (s, 3H). ¹³C NMR (151 MHz, CDCl₃) δ 165.78, 163.20, 139.79, 131.55, 123.31, 122.69, 113.50, 60.41, 58.50, 56.71, 56.15, 55.42, 50.06, 42.33, 39.76, 39.53, 38.30, 37.07, 36.67, 36.20, 35.81, 31.95, 31.90, 28.25, 28.03, 27.95, 24.31, 23.84, 22.83, 22.58, 21.06, 19.40, 18.73, 18.46, 14.21, 11.87.

Note: The main components of the crude compound of formula (7-5) are the compounds (7-5) and (7'-5), and the ratio is the compounds (7-5)/(7'-5)≈92/8.

### 6. Preparation of the compound of formula (7-6)

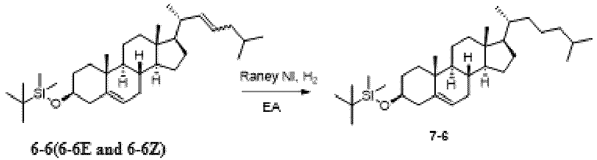

Ethyl acetate (75mL), the crude compound of formula (6-6) (10g, 20.04mmol) were added to a flask, stirred to dissolve and be clarified, then added with Raney Ni (10g, wet weight, activated according to the standard procedure), H₂ (1atm), and reacted for 15 h at 35°C, after the completion of the reaction was monitored by gas phase, filtered to remove Raney Ni through diatomaceous earth. The filtrate was concentrated under reduced pressure to afford the crude compound of formula (7-6) as a white solid, which was directly used in the purification step. The white solid was added to a mixed solution of methanol and ethyl acetate, heated reflux until dissolved and clarified, naturally cooled to 15°C, stirred for 4h, and suction filtered to afford the compound of formula (7-6) (8.25g of white solid, molar yield of 82.5%, gas chromatographic purity of 98.3%). ¹H NMR (500 MHz, CDCl₃) δ 5.43 - 5.30 (m, 1H), 3.53 - 3.47 (m, 1H), 2.32 - 2.26 (m, 1H), 2.21 - 2.17 (m, 1H), 2.07 - 1.96 (m, 2H), 1.89 - 1.80 (m, 2H), 1.77 - 1.70 (m, 1H), 1.62 - 1.45 (m, 8H), 1.41 - 1.31 (m, 3H), 1.31 - 1.24 (m, 1H), 1.18 - 1.06 (m, 7H), 1.02 (s, 4H), 1.01 - 0.98 (m, 1H), 0.93 (d, *J* = 6.4 Hz, 3H), 0.91 (s, 9H), 0.89 (dd, *J =* 6.6, 2.4 Hz, 6H), 0.69 (s, 3H), 0.08 (s, 6H). ¹³C NMR (151 MHz, CDCl₃) δ 141.60, 121.19, 72.67, 56.82, 56.16, 50.23, 42.84, 42.34, 39.82, 39.53, 37.40, 36.61, 36.20, 35.79, 32.10, 31.96, 31.92, 28.25, 28.03, 25.96, 24.31, 23.82, 22.83, 22.57, 21.08, 19.44, 18.73, 18.28, 11.86.

Note: The main components of the crude compound of formula (7-6) are the compounds (7-6) and (7'-6), and the ratio is the compounds (7-6)/(7'-6)≈92/8.

### Example 8 Preparation of cholesterol by hydrolysis of the compound of formula (7)

### 1. Preparation of cholesterol by hydrolysis of the compound of formula (7-1)

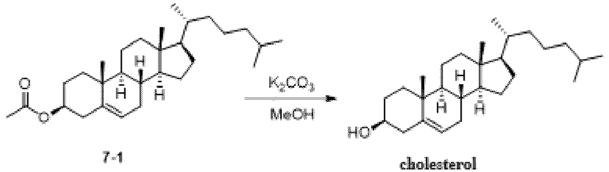

Methanol (43mL) and K₂CO₃ (2.07g, 15mmol) were added to a flask, dissolved and clarified, then added with the compound of formula (7-1) (4.28g, 10mmol) under the protection of N₂, heated to 65°C and reacted for 2h, after the completion of the reaction was monitored by TLC, cooled to 25°C, added with 2mol/L diluted hydrochloric acid to adjust the pH to 7-8, evaporated to remove methanol under reduced pressure, added with water (20mL), stirred for 2h at 25°C, and suction filtered; water was added to the filter cake ( 20 mL), stirred at 25°C for 2 h, suction filtered, and dried to afford refined cholesterol (3.80 g of white solid, molar yield of 98.45%, gas chromatography purity of 99.38%, Figure 6).

### 2. Preparation of cholesterol by hydrolysis of the compound of formula (7-2)

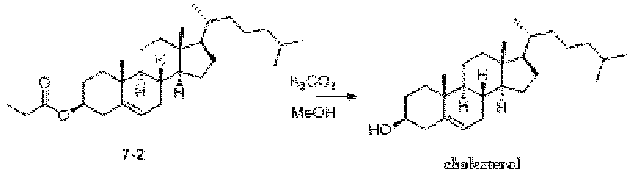

Methanol (50mL) and K₂CO₃ (2.07g, 15mmol) were added to a flask, dissolved and clarified, then added with the compound of formula (7-2) (4.4g, 10mmol) under the protection of N₂, heated to 65°C and reacted for 2h, after the completion of the reaction was detected by TLC, cooled to 25°C, added with 2mol/L diluted hydrochloric acid to adjust the pH to 7-8, evaporated to remove methanol under reduced pressure, added with water (20mL), stirred for 2h at 25°C, and suction filtered; water was added to the filter cake ( 20ml), stirred for 2h at 25°C, suction filtered, and dried to afford refined cholesterol (3.81g of white solid, molar yield of 98.7%, gas chromatography purity of 99.0%).

### 3. Preparation of cholesterol by hydrolysis of the compound of formula (7-3)

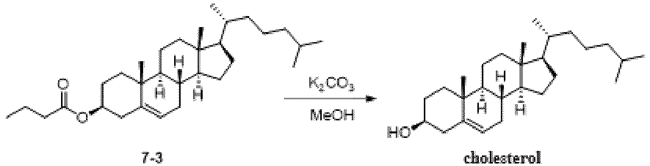

Methanol (45mL) and K₂CO₃ (1.8g, 13mmol) were added to a flask, dissolved and clarified, then added with the compound of formula (7-3) (4.56g, 10mmol) under the protection of N₂, heated to 65°C and reacted for 2h, after the completion of the reaction was monitored by TLC, cooled to 25°C, added with 2mol/L diluted hydrochloric acid to adjust the pH to 7-8, evaporated to remove methanol under reduced pressure, added with water (20mL), stirred for 2h, at 25°C and suction filtered; water was added to the filter cake ( 20 mL), stirred for 2 h at 25°C, suction filtered, and dried to afford refined cholesterol (3.8 g of white solid, molar yield of 98.5%, gas chromatography purity of 99.3%).

### 4. Preparation of cholesterol by hydrolysis of the compound of formula (7-4)

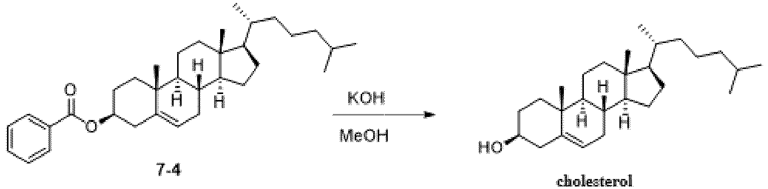

Methanol (50mL) and KOH (0.62g, 11mmol) were added to a flask, dissolved and clarified, then added with the compound of formula (7-4) (4.9g, 10mmol) under the protection of N₂, and reacted for 6h at 45°C, after the completion of the reaction was detected by TLC, cooled to 25°C, added with 2mol/L diluted hydrochloric acid to adjust PH = 5-6, evaporated to remove methanol under reduced pressure, added with water (20ml), stirred for 2h at 25°C, suction filtered; 80% ethanol (10ml) was added to the filter cake, stirred for 2 h at 25°C, suction filtered, and dried to affrd refined cholesterol (3.66 g of white solid, molar yield 94.8%, gas chromatography purity of 98.9%).

### 5. Preparation of cholesterol by hydrolysis of the compound of formula (7-5)

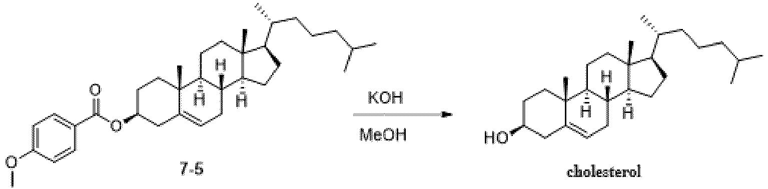

Methanol (60mL) and KOH (0.62g, 11mmol) were added to a flask, dissolved and clarified, then added with the compound of formula (7-5) (5.2g, 10mmol) under the protection of N₂, and reacted for 8h at 45°C, after the completion of the reaction was detected by TLC, cooled to 25°C, added with 2mol/L diluted hydrochloric acid to adjust PH = 5-6, evaporated to remove methanol under reduced pressure, addde with water (20ml), stirred for 2h at 25°C, suction filtered; 80% ethanol (10ml) was added to the filter cake, stirred for 2 h at 25°C, suction filtered, and dried to afford refined cholesterol (3.61 g of white solid, molar yield of 93.5%, gas chromatography purity of 98.7%).

### 6. Preparation of cholesterol by deprotection of the compound of formula (7-6)

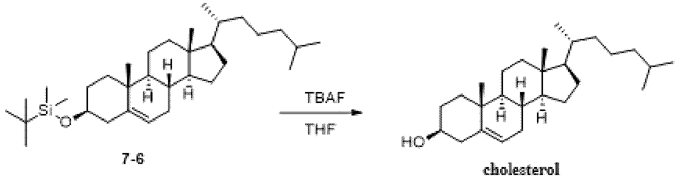

Tetrahydrofuran (25mL), the compound of formula (7-6) (5.01g, 10mmol) were added to a flask, dissolved and clarified, then added with TBAF.3H₂O (15.7g, 50mmol) under the protection of N₂, and reacted for 24h at 25°C, after the completion of the reaction was monitored by TLC, added with saturated ammonium chloride to quench, evaporated to remove tetrahydrofuran under reduced pressure, added with dichloromethane (150mL) and water (120mL) for extraction, separated. The organic phase was washed with saturated NaHCO₃ aqueous solution, 2N dilute hydrochloric acid, water, saturated NaCl in sequence, concentrated under reduced pressure to afford crude cholesterol. 80% Ethanol (10ml) was added to the filter cake, slurried for 2h at 25°C, and suction filtered to afford refined cholesterol (3.68g of white solid, molar yield of 95.3%, gas chromatography purity of 98.56%).

### Comparative example 1

The patent document (background technology Scheme 2, CN 105218610 A) reports that cholesterol is synthesized with a total molar yield of 67% through 5 steps of reaction using the degradation product of stigmasterol as a raw material. In the technical route of this patent document, the first step reaction is shown in the following reaction formula 1:

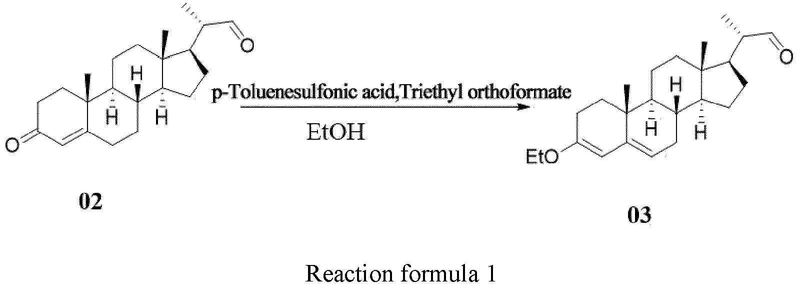

The patent document CN 105218610 A describes that reaction formula 1 uses the compound of formula (02) after BA oxidation as a raw material, and ethanol as a solvent, keeps at 40°C and reacts for 4 hours under the action of p-toluenesulfonic acid and triethyl orthoformate to afford compound of formula (03) (molar yield of 97.50%).

According to the experimental method provided by the above-mentioned patent document, the present invention uses ethanol as a solvent, and the compound of formula (02) as a substrate, under the catalysis of p-toluenesulfonic acid and triethyl orthoformate, keeps at 40°C and reacts for 4 hours, and TLC detects that the raw material has fully reacted, and the compound of formula (03') (shown in reaction formula 2) is obtained by post-processing according to the method of the patent document (CN 105218610 A), which is inconsistent with the compound described in the patent document. The present invention also attempts to reduce the amount of triethyl orthoformate, and TLC detects that the raw material reacts completely, but does not obtain the result of reaction formula 1 described in the patent document (CN 105218610 A), but obtains the result shown in the following reaction formula 3. It shows that when the 3-carbonyl group of the compound of formula (02) is protected according to the method reported in patent document CN 105218610 A, the C-22 aldehyde group will be preferentially protected to generate acetal, resulting in the compounds of formula (03 ') and (03 ") as shown in reaction formula 2 or reaction formula 3, but the compound of formula (03) as described in the patent document (CN 105218610 A) can not be obtained, obviously, the compounds of formula (03 ') and (03 ") as shown in reaction formula 2 or reaction formula 3 cannot be subjected to subsequent Wittig reactions.

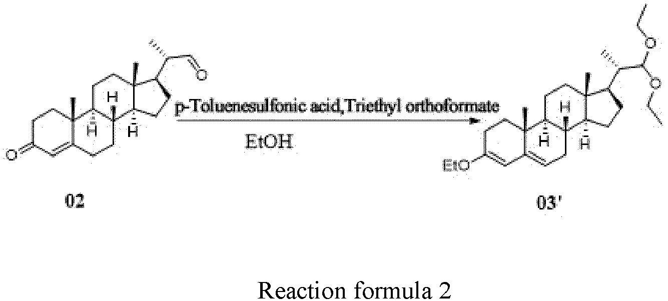

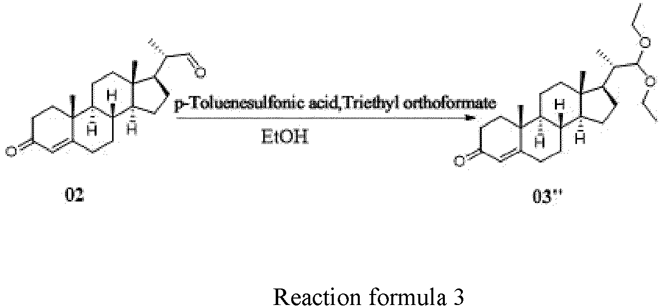

Experimental method: Ethanol (4ml), triethyl orthoformate (2ml), the compound of formula (02) (2.00g, 70.20mmol) and p-toluenesulfonic acid (20mg, 0.12mmol) were added to a flask, kept at 40°C and reacted for 4 hours, and TLC detected that the raw material had reacted completely. Sodium acetate (20mg) was added in an ice bath, the filter cake was washed with water until the eluate was neutral, after being drained, purified by column chromatography (petroleum ether: ethyl acetate = 20: 1) to afford the compound of formula (03') (2.30g of colorless oily substance, molar yield of 88%). ¹H NMR (600 MHz, CDCl₃) *δ* 5.23 - 5.19 (m 1H), 5.10 (d, *J =* 1.9 Hz, 1H), 4.30 (d, *J* = 2.4 Hz, 1H), 3.81 - 3.70 (m, 4H), 3.61 - 3.55 (m, 1H), 3.49 - 3.41 (m, 2H), 2.31 - 2.25 (m, 1H), 2.18 - 1.96 (m, 4H), 1.85 - 1.78 (m, 2H), 1.71 - 1.52 (m, 7H), 1.43 - 1.33 (m, 4H), 1.29 (d, *J =* 7.0 Hz, 3H), 1.22 - 1.18 (m, 6H), 0.99 (d, *J =* 6.7 Hz, 3H), 0.96 (s, 3H), 0.70 (s, 3H).¹³C NMR (150 MHz, CDCl₃) *δ* 154.52, 141.06, 118.03, 106.07, 99.04, 64.60, 63.35, 62.14, 56.48, 51.88, 48.33, 42.59, 40.39, 39.62, 35.18, 33.86, 31.92, 31.86, 27.72, 25.55, 24.42, 21.17, 18.96, 15.49, 15.37, 14.68, 11.94, 11.89. HRMS(ESI): calcd for C₂₈H₄₆NaO₃ [M+Na]⁺,453.3339, found 453.3328.

Experimental method: Ethanol (4ml), triethyl orthoformate (1ml), the compound of formula (02) (2.00g, 70.20mmol) and p-toluenesulfonic acid (20mg, 0.12mmol) were added to a flask, kept at 40°C and reacted for 4 hours, and TLC detected that the raw materials had reacted completely. Sodium acetate (20mg) was added in an ice bath, the filter cake was washed with water until the eluate was neutral, after being drained, purified by column chromatography (petroleum ether: ethyl acetate = 5:1) to afford the compound of formula (03") (2.32g of colorless oil, molar yield of 95%). ¹H NMR (600 MHz, CDCl₃) *δ* 5.69 (d, *J* = 1.8 Hz, 1H), 4.27 (d, *J =* 2.4 Hz, 1H), 3.78 - 3.72 (m, 1H), 3.58-3.53 (m, 1H), 3.47 - 3.40 (m, 2H), 2.42 - 2.21 (m, 4H), 2.01 - 1.97 (m, 2H), 1.84 - 1.77 (m, 2H), 1.69 - 1.58 (m, 3H), 1.53 - 1.47 (m, 2H), 1.41 - 1.27 (m, 3H), 1.21 - 1.17 (m, 6H), 1.16 (d, *J* = 4.1 Hz, 4H), 1.13 - 1.08 (m, 1H), 1.04 - 0.98 (m, 2H), 0.96 (d, *J =* 6.8 Hz, 3H), 0.93 - 0.86 (m, 1H), 0.69 (s, 3H).¹³C NMR (150 MHz, CDCl₃) *δ* 199.58, 171.53, 123.76, 105.95, 64.58, 63.40, 55.39, 53.81, 51.81, 42.52, 40.33, 39.42, 35.69, 35.63, 33.97, 32.92, 32.03, 27.62, 24.37, 21.01, 17.37, 15.48, 15.36, 11.91, 11.86.

### Comparative example 2

The second Wittig reaction reported in patent document (CN 105218610 A) is shown in the following reaction formula 4:

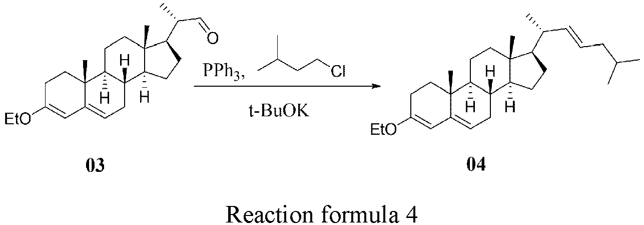

In this patent document, toluene was used as a solvent, added with triphenylphosphine and 1-chloro-3-methylbutane to react under reflux for 2h, added with potassium tert-butoxide and the compound of formula (03), to react under reflux for 4h to afford the compound of formula (04 ), molar yield: 90.29%. If the present invention adopts the method of patent document (CN 105218610 A) (comparative example 2, reaction formula 4), using 1-chloro-3-methylbutane or 1-bromo-3-methylbutane to be subjected to Witting reaction on the compound of formula (2) involved in the present invention, triphenylphosphine and 1-chloro-3-methylbutane were refluxed for 2h. The result of the reaction is shown in reaction formula 5, and the compound of formula (3') is obtained, but the expected compound of formula (3) is not obtained, indicating that the method disclosed in the patent document (CN 105218610 A) cannot be applied to the present invention.

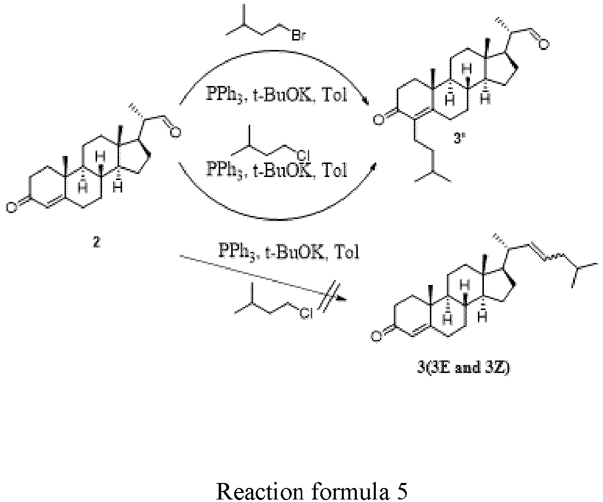

Experimental method: Triphenylphosphine (797mg, 3.04mmol), 1-chloro-3-methylbutane (324mg, 3.04mmol), toluene (10mL) were added to a flask, reacted under reflux for 2h, cooled to 25°C, added with potassium tert-butoxide (307mg, 2.74.02mmol) in three batches in an ice bath, stirred in ice bath for 0.5h, then added with the compound of formula (2) (500mg, 1.52mmol) and reacted under reflux for 4h. After the completion of the reaction was monitored by TLC, cooled to 25°C, added with 2M HCl (4mL) to adjust the solution to be neutral, extracted with dichloromethane (50mL) and water (50mL). The organic phase was washed with water and saturated brine in sequence, dried with anhydrous sodium sulfate, concentrated under reduced pressure, and purified by column chromatography (PE:EA=20:1) to afford the compound of formula (3') (516 mg of colorless oil, molar yield of 85%). ¹H NMR (600 MHz, CDCl₃) *δ* 9.53 (d, *J =* 5.0 Hz, 1H), 2.72 - 2.68 (m, 1H), 2.39 - 2.25 (m, 6H), 2.10 (dd, *J =* 14.4, 4.8 Hz, 1H), 1.94 - 1.86 (m, 3H), 1.68 - 1.53 (m, 8H), 1.43 - 1.30 (m, 4H), 1.25 (s, 3H), 1.15 (s, 3H), 1.04 (d, *J =* 6.7 Hz, 3H), 0.89 (d, *J =* 6.6 Hz, 6H), 0.72 (s, 3H).¹³C NMR (150 MHz, CDCl₃) *δ* 205.71, 198.54, 163.30, 133.32, 55.49, 54.31, 51.82, 48.85, 42.21, 39.05, 38.94, 38.40, 35.35, 35.16, 33.95, 32.12, 29.71, 28.43, 27.31, 26.49, 23.74, 23.21, 22.54, 20.74, 17.80, 13.60, 12.97. HRMS(ESI): calcd for C₂₇H₄₂NaO₂ [M+Na]⁺,421.3077, found 421.3075.

Triphenylphosphine (797mg, 3.04mmol), 1-bromo-3-methylbutane (459mg, 3.04mmol), toluene 10mL were added to a flask, reacted under reflux for 2h, cooled to 25°C, added with potassium tert-butoxide (307mg, 2.74 mmol) in batches in an ice bath, stirred in an ice bath for 0.5h, then added with the compound of formula (2) (500mg, 1.52mmol) and reacted under reflux for 2.5h. After the completion of the reaction was monitored by TLC, cooled to 25°C, added with 2M HCl (4mL) to adjust the solution to be neutral, extracted with dichloromethane (50mL) and water (50mL). The organic phase was washed with water and saturated brine in sequence, dried with anhydrous sodium sulfate, concentrated under reduced pressure, and purified by column chromatography (PE:EA=20:1) to afford the compound of formula (3') (534 mg of colorless oil, molar yield of 88%).

### Comparative example 3

In the present invention, the 3-position ester group of the compound of formula (4) is reduced by NaBH₄ to obtain the compound of formula (5), and then the compound of formula (5) is directly subjected to selectively hydrogenated reduction or subjected to hydroxyl protection, selective hydrogenated reduction, deprotection or hydrolysis reaction to obtain cholesterol. In addition, we tried to use Raney Ni/H₂ as a reducing agent to carry out selective hydrogenation reduction reaction on the double bond of the side chain, and then use NaBH₄ to reduce the 3-position ester group. The result of the reaction is shown in reaction formula 6, the compounds of formula (6-1) and (6'-1) were obtained, but the expected compound of formula (9) was not obtained, indicating that Raney Ni/H₂ as the reducing agent cannot selectively hydrogenate and reduce the side chains, therefore, the reaction sequence of the present invention cannot be changed.

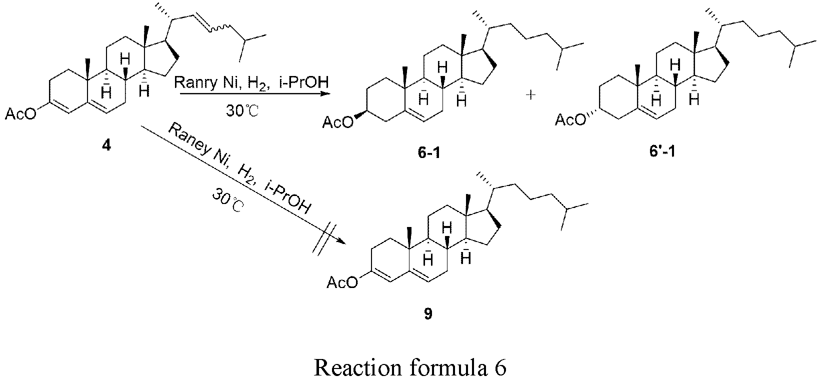

Experimental method: The compound of formula (4) (2.10g, 17.2mmol), Raney Ni (4.20g, wet weight), isopropanol (30mL), H₂ (1atm) was added to a flask, reacted for 11h at 30°C, after the completion of the reaction was monitored by TLC, filtered to remove Raney Ni. The filtrate was concentrated under reduced pressure to afford a mixture of formula (6-1) and formula (6'-1). According to ¹H NMR results of the product, the ratio of the compounds of formula (6-1) to isomer (6'-1) is 1:0.28.

### Comparative example 4

In the present invention, the crude compound of formula (6-1) is reduced by Raney Ni/H₂ to synthesize the crude compound of formula (7-1). As shown in the following reaction formula 7:

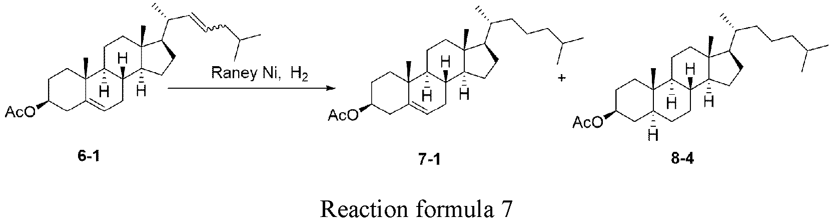

The present invention uses H₂ as a reducing agent, Raney Ni as a catalyst to carry out selectively hydrogenation reduction on the double bond of the side chain, and the content of the overreduced impurity compound of formula (8-4) in the crude compound of formula (7-1) obtained is relative low, less than 0.7% (judged by ¹H NMR), which is easy to be removed by purification; at the same time, when trying to use H₂ as the reducing agent and 10% Pd/C as the catalyst to carry out selective hydrogenation reduction on the double bond o the side chain, the content of the overreduced impurity compound of formula (8-4) in the crude compound of formula (7-1) obtained is more than 5% (judged by ¹H NMR), which is difficult to be removed by purification, and fails to achieve the expected goal. These results indicate that 10% Pd/C has relatively high catalytic activity and poor selectivity, and cannot replace Raney Ni as the catalyst for selective hydrogenation reduction on side chains.

Experimental method: The compound of formula (6-1) (1.50g, 3.56mmol), 10% Pd/C (150mg), ethyl acetate (30mL), H₂ (1atm) were added to a flask, reacted for 9h at 30°C, after the completion of the reaction was monitored by TLC, filtered to remove Pd/C, and the filtrate was concentrated under reduced pressure. According to ¹H NMR spectrum of the product, the content of the overreduced impurity compound of formula (8-4) is greater than 5%.

The protection content of the present invention is not limited to the above embodiments. Without departing from the spirit and scope of the concept of the present invention, changes and advantages conceivable by those skilled in the art are all included in the present invention, and the appended claims are the protection scope.

## Claims

1. A method for synthesizing cholesterol with plant-derived 21-hydroxy-20-methylpregn-4-en-3-one BA as raw material, wherein, the method uses BA as raw material to synthesize the cholesterol through oxidation, Wittig reaction, acetylation, reduction and selective hydrogenation reduction; or synthesis of the cholesterol by oxidation, Wittig reaction, acetylation, reduction, hydroxyl protection, selective hydrogenation reduction, deprotection or hydrolysis, specifically comprising the following steps:
step (a), in the first solvent, the BA shown in formula (1) undergoes oxidation reaction to obtain the compound of formula (2);
step (b), in the second solvent, the compound of formula (2) undergoes Wittig reaction to obtain the compound of formula (3);
step (c), in the third solvent, the compound of formula (3) undergoes acetylation reaction to obtain the compound of formula (4);
step (d), in the fourth solvent, the compound of formula (4) undergoes reduction reaction to obtain the compound of formula (5);
step (e), in the fifth solvent, the compound of formula (5) undergoes selective hydrogenation reduction reaction to obtain cholesterol;
or,
step (f), in the sixth solvent, the compound of formula (5) undergoes hydroxyl protection reaction to obtain the compound of formula (6);
step (g), in the seventh solvent, the compound of formula (6) undergoes selective hydrogenation reduction reaction to obtain the compound of formula (7);
step (h), in the eighth solvent, the compound of formula (7) undergoes deprotection or hydrolysis reaction to obtain cholesterol;
wherein, the reaction process of the said method is as shown in route (A): 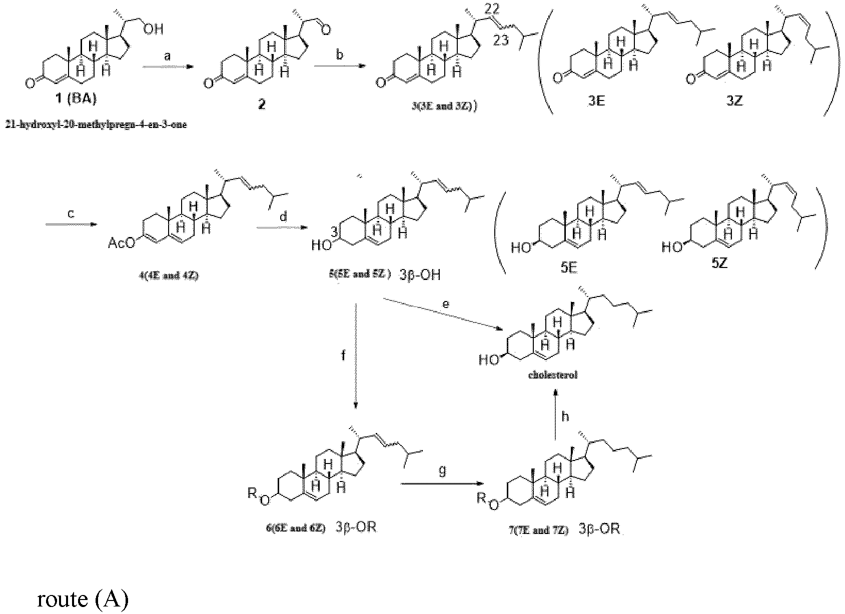
wherein, R is selected from ester group, silicon ether group.

2. The method according to claim 1, wherein, the ester group is selected from one or more of C2-C10 straight chain ester group, isobutyl ester 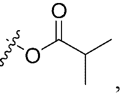 isopentyl ester 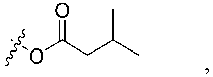 phenyl ester 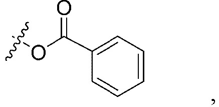 p-methoxyphenylcarboxylate 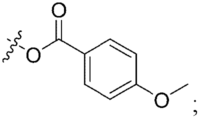 the silicon ether group is selected from one or both of trimethylsilyl ether group 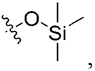 tert-butyldimethylsilyl ether group

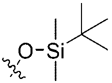

3. The method according to claim 1, wherein, in step (a), the said oxidation reaction is specifically: in the first solvent, BA shown in formula (1), TEMPO, sodium bicarbonate, tetrabutylammonium bromide and oxidizing agent are subjected to oxidation reaction, to obtain the compound of formula (2).

4. The method according to claim 3, wherein, the mol ratio of BA shown in formula (1), TEMPO, sodium bicarbonate, tetrabutylammonium bromide, and oxidizing agent is 1: (0~1): (0~20): (0~1): (1~5); and/or, the said oxidizing agent is selected from one or more of N-chlorosuccinimide NCS, N-bromosuccinimide NBS, 2-iodoacylbenzoic acid IBX; and/or, the said first solvent is selected from one or more of dichloromethane, tetrahydrofuran, toluene, dimethyl sulfoxide, water; and/or, the temperature of the said oxidation reaction is 0 ~ 30°C; and/or, the time of the said oxidation reaction is 3~8 hours.

5. The method according to claim 1, wherein, in step (b), the said Wittig reaction is specifically: 1-halo-3-methylbutane and triphenylphosphine are added into the second solvent, reflux reaction, cooling, suction filtration, to obtain quaternary phosphonium salt; then added the said quaternary phosphonium salt, potassium tert-butoxide into the second solvent, and then added the compound of formula (2), and subjected to Wittig reaction, to obtain the compound of formula (3) .

6. The method according to claim 5, wherein, the mol ratio of the compound of formula (2), 1-halo-3-methylbutane, triphenylphosphine, and potassium tert-butoxide is 1:(1~4): (1~4): (1~4); and/or, the said second solvent is selected from one or more of xylene, toluene, benzene, tetrahydrofuran, heptane; and/or, the said 1-halo-3-methylbutane is selected from one or both of 1-chloro-3-methylbutane and 1-bromo-3-methylbutane; and/or, the temperature of the said Wittig reaction is -10~ 145°C; and/or, the time of the said Wittig reaction is 0.5~24 hours.

7. The method according to claim 1, wherein, in step (c), the said acetylation reaction is specifically: the compound of formula (3), acetyl chloride, acetic anhydride, and alkali are subjected to acetylation reaction in the third solvent to obtain the compound of formula (4).

8. The method according to claim 7, wherein, the molar ratio of the compound of formula (3), acetyl chloride, acetic anhydride, and alkali is 1: (0.5 ~ 62.5): (1 ~ 62.5): (0 ~ 6 ); and/or, the said alkali is selected from one or more of pyridine, triethylamine, DIPEA, DMAP, diisopropylamine; and/or, the said third solvent is selected from acetic anhydride, acetyl chloride, ethyl acetate, dichloromethane; and/or, the temperature of the said acetylation reaction is 40 ~ 110°C; and/or, the time of the said acetylation reaction is 1~10 hours.

9. The method according to claim 1, wherein, in step (d), the said reduction reaction is specifically: the compound of formula (4) and the reducing agent are subjected to reduction reaction in the fourth solvent to obtain the compound of formula (5).

10. The method according to claim 9, wherein, the molar ratio of the compound of formula (4) and the reducing agent is 1:(1 ~25); and/or, the said fourth solvent is selected from one or more of tetrahydrofuran, ethanol, water, dichloromethane, 2-methyltetrahydrofuran, isopropanol, acetic acid, methyl tert-butyl ether; and/or, the said reducing agent is selected from one or both of NaBH₄ and KBH₄; and/or, the temperature of the said reduction reaction is 0 ~ 50°C; and/or, the time of the said reduction reaction is 6~12 hours.

11. The method according to claim 1, wherein, in step (e), the said selective hydrogenation reduction reaction is specifically: under the action of a catalyst, the compound of formula (5) and reducing agent are subjected to selective hydrogenation reduction reaction in the fifth solvent to obtain cholesterol.

12. The method according to claim 11, wherein, the said reducing agent is selected from H₂; and/or, the said catalyst is Raney Ni; and/or, the mass ratio of the compound of formula (5) and the catalyst is 1: (0.05~5); and/or, the said fifth solvent is selected from one or more of isopropanol, dichloromethane, methanol, 2-methyltetrahydrofuran, tetrahydrofuran, ethanol, water, methyl tert-butyl ether, ethyl acetate, toluene; and/or, the temperature of the said hydrogenation reduction reaction is 0~60°C; and/or, the pressure of reducing agent H₂ in the hydrogenation reduction reaction is 1~20 atm; and/or, the time of the said hydrogenation reduction reaction is 6~10 hours.

13. The method according to claim 1, wherein, in step (f), the said hydroxyl protection reaction is specifically: the compound of formula (5), under the action of an alkali, reacts with the reagent for protecting hydroxyl in the six solvent to obtain the compound of formula (6).

14. The method according to claim 13, wherein, when R is an ester group, the said sixth solvent is selected from one or more of ethyl acetate, dichloromethane, chloroform, DMF, toluene, tetrahydrofuran, 2-methyltetrahydrofuran; and/or, the said alkali is selected from one or both of triethylamine, diisopropyl ethylamine, imidazole, pyridine, DMAP; and/or, the molar ratio of the compound of formula ( 5), the reagent for protecting hydroxyl, and the alkali is 1:(1~4):(0.05~5); and/or, the temperature of the said reaction is 0~50°C; and/or, the time of the said hydroxyl group protection reaction is 2~24 hours;
when R is a silicon ether group, the said sixth solvent is selected from one or more of DMF, dichloromethane, chloroform, tetrachloromethane; and/or, the said alkali is selected from one or more of triethylamine, diisopropyl ethylamine, imidazole, pyridine, DMAP; and/or, the molar ratio of the compound of formula (5), the reagent for protecting hydroxyl, alkali is 1:(2~4): (4-8); and/or, the temperature of the said hydroxyl protection reaction is 0 ~ 50°C; and/or, the time of the said hydroxyl protection reaction is 2~24 hours.

15. The method according to claim 1, wherein, in step (g), the said selective hydrogenation reduction reaction is specifically: under the action of a catalyst, the compound of formula (6), and the reducing agent are subjected to selective hydrogenation reduction reaction in the seventh solvent to obtain the compound of formula (7).

16. The method according to claim 15, wherein, the said reducing agent is selected from H₂; and/or, the catalyst is Raney Ni; and/or, the mass ratio of the compound of formula (6) and the catalyst is 1: (0.05 ~ 5); and/or, the said seventh solvent is selected from one or more of 2-methyltetrahydrofuran, tetrahydrofuran, ethyl acetate, toluene, isopropanol; and/or, the temperature of the said hydrogenation reduction reaction is 0 ~ 60°C; and/or, the pressure of the reducing agent H₂ in the said hydrogenation reduction reaction is 1 ~ 20 atm; and/or, the time of the said hydrogenation reduction reaction is 4~48 hours.

17. The method according to claim 1, wherein, when R is an ester group, in step (h), the hydrolysis reaction is specifically: under the action of an alkali , the compound of formula (7) is subjected to hydrolysis reaction in the eighth solvent to obtain cholesterol.

18. The method according to claim 17, wherein, the said alkali is selected from one or more of LiOH, KOH, NaOH, t-BuOK, K₂CO₃; and/or, the molar ratio of the compound of formula (7) and alkali is 1: (0.5 ~2); and/or, the said eighth solvent is selected from one or both of methanol and ethanol; and/or, the temperature of the said hydrolysis reaction is 10~75°C; and/or, the time of the said hydrolysis reaction is 0.3~12 hours.

19. The method according to claim 1, wherein, when R is a silicon ether group, in step (h), the deprotection reaction is specifically: under the action of a catalyst, the compound of formula (7) is subjected to deprotection reaction in the eighth solvent to obtain cholesterol.

20. The method according to claim 19, wherein, the said catalyst is selected from one or more of tetrabutylammonium fluoride TBAF, tetrabutylammonium fluoride trihydrate TBAF 3H₂O, boron trifluoride ether, acetic acid, hydrogen chloride in the ethyl acetate solution; and/or, the mass ratio of the compound of formula (7) and the catalyst is 1:(1~6); and/or, the said eighth solvent is selected from one or both of tetrahydrofuran, water; and/or, the temperature of the said deprotection reaction is 10 ~ 75°C; and/or, the time of the said deprotection reaction is 2 ~ 48 hours.

21. Compounds, wherein, the structure of the compounds are shown as formula 6-2(E), 6-3(E), 6-5(E), 6-6(E), 6-2(Z), 6-3(Z), 6-5(Z), 6-6(Z), 6'-1(E), 6'-2(E), 6'-3(E), 6'-4(E) , 6'-5(E), 6'-6(E), 6'-1(Z), 6'-2(Z), 6'-3(Z), 6'-4(Z), 6 '-5(Z), 6'-6(Z):
